# EUROPEAN PATENT SPECIFICATION

(11) **EP 4 233 708 B1**
(45) Date of publication and mention of the grant of the patent: **01.01.2025**
(21) Application number: 23157886.5
(22) Date of filing: 22.02.2023
(51) Int. Cl.: A61B 5/08, A61B 5/11, A61B 5/113, A61B 5/00

(54) **SYSTEM AND METHODS FOR ANALYZING RESPIRATORY FUNCTION USING GUIDED BREATHING**
SYSTEM UND VERFAHREN ZUR ANALYSE DER ATEMFUNKTION UNTER GEFÜHRTER ATMUNG
SYSTÈME ET PROCÉDÉS D'ANALYSE DE FONCTION RESPIRATOIRE À L'AIDE D'UNE RESPIRATION GUIDÉE

(30) Priority: 23.02.2022 US 202263313118 P; 10.02.2023 US 202318108326
(43) Date of publication of application: 30.08.2023
(73) Proprietor: Apple Inc., Cupertino, CA 95014 (US)
(72) Inventor: CHATTERJEE, Anirban, Cupertino, CA, 95014 (US); VALSAN, Gopal, Cupertino, CA, 95014 (US); SUMANAWEERA, Thilaka S., Cupertino, CA, 95014 (US)
(74) Representative: Lang, Johannes

(56) References cited:
- WO-A1-2013/046094
- CN-A- 113 712 519
- US-A1- 2017 354 795
- MASSARONI CARLO ET AL: "Contactless Methods For Measuring Respiratory Rate: A Review", IEEE SENSORS JOURNAL, IEEE, USA, vol. 21, no. 11, 11 September 2020 (2020-09-11), pages 12821 - 12839, XP011857227, ISSN: 1530-437X, [retrieved on 20210527], DOI: 10.1109/JSEN.2020.3023486

## Description

### CROSS-REFERENCE TO RELATED APPLICATION

This application is a nonprovisional and claims the benefit under 35 U.S.C. § 119(e) of U.S. Provisional Patent Application No. 63/313,118, filed February 23, 2022.

### FIELD

The described embodiments relate generally to systems and methods for analyzing the respiratory function of a user. More particularly, the present embodiments relate to systems and methods that use guided breathing to determine respiratory health parameters of a user.

### BACKGROUND

Individuals with chronic obstructive pulmonary disease (COPD), asthma, or other airway related conditions may experience reduced air flow when breathing (also known as "airway obstruction") that make it more difficult to breath. This airway obstruction may range in severity and may also change over time. Spirometry is a current breathing test that is often used to assess an individual's lung function and potential airway obstruction. During a spirometry test, an individual breathes into a measurement device, which measures air flow metrics that are used to determine an individual's lung function. The accuracy and/or repeatability of spirometry measurements depends on how closely an individual can follow the test protocol. Accordingly, in some cases, the accuracy and/or repeatability of spirometer measurements are less than desirable. Moreover, it may be difficult for individuals to perform accurate and/or repeatable spirometer measurements without oversight from a clinician.

### SUMMARY

The invention is directed to a system for measuring respiratory function of a user. The system includes an optical sensing unit that is configured to identify a torso of the user, detect movement of the torso with respect to the optical sensing unit, and output one or more signals indicative of the movement of the torso of the user. The system includes an electronic device configured to provide a first request for the user to breathe at a first rate during a first time period and a second request for the user to breathe at a second rate during a second time period. The system also includes a processing unit programmed to receive first signals generated during the first time period based on the movement of the torso and determine a first respiration parameter using the first signals. The processing unit is programmed to receive second signals generated during the second time period based on the movement of the torso and determine a second respiration parameter using the second signals. The processing unit determines a level of respiratory function based on the first respiration parameter and the second respiration parameter.

Embodiments are also directed to a system for analyzing respiratory function of a user. The system can include a sensing unit configured to detect movement of a torso of the user and output one or more signals indicative of the detected movement of the torso. The system can include an electronic device configured to provide a request for the user to breathe at a first rate during a first time period. The system can also include a processing unit that is programmed to receive the one or more signals, determine a first respiration parameter based on detected movement of the torso during the first time period, where the first respiration parameter is associated with the first breathing rate. The processing unit can be programmed to determine a second respiration parameter based on detected movement of the torso during a second time period, the second respiration parameter associated with a second breathing rate. The processing unit can determine a respiratory metric using the first respiration parameter and the second respiration parameter.

The invention is further directed to methods for measuring respiratory function of a user. The methods include outputting, from an electronic device, a first request for the user to breathe at a first rate for a first time period and obtaining, by an optical sensing unit, a first set of respiratory measurements during the first time period, the first set of respiratory measurements including distance measurements corresponding to movement of a torso of the user. The methods include outputting, from the electronic device, a second request for the user to breathe at a second rate for a second time period and obtaining, by the optical sensing unit, a second set of respiratory measurements during the second time period, the second set of respiratory measurements including distance measurements corresponding to movement of the torso of the user. The methods include determining, by a processing unit, a respiratory metric for the user based on the first and second sets of respiratory measurements.

### Prior Art

From document CN113712519A, a system is known for measuring respiratory parameters by PPG at different target respiratory rates, and for combining these to determine a respiratory metric.

### BRIEF DESCRIPTION OF THE DRAWINGS

The disclosure will be readily understood by the following detailed description in conjunction with the accompanying drawings, wherein like reference numerals designate like structural elements, and in which:
FIG. 1 shows an example system for determining respiratory function of a user;
FIG. 2 shows an example process for determining respiratory function of a user;
FIGs. 3A and 3B show an example chest movement analysis for determining respiratory parameters of a user;
FIG. 4 shows an example signal flow between different devices of a respiratory monitoring system;
FIG. 5 shows an example process for performing an enrollment session for respiratory sensing;
FIG. 6 shows an example process for performing a respiratory sensing session for a user;
FIGs. 7A and 7B show examples of chest movement data measured at two different breathing rates;
FIG. 8 shows an example analysis for determining a breathing metric for a user;
FIG. 9 shows an example process for determining a respiratory parameter based on differences between a user's actual breathing rate and a requested breathing rate;
FIGs. 10A-10F show examples of a user output from an electronic device that can be used to guide a user through a requested breathing profile;
FIGs. 11A-11F show examples of a user output from an electronic device that can be used to guide a user through a requested breathing profile and provide user feedback; and
FIG. 12 is an example block diagram of a respiratory monitoring system.

It should be understood that the proportions and dimensions (either relative or absolute) of the various features and elements (and collections and groupings thereof) and the boundaries, separations, and positional relationships presented therebetween, are provided in the accompanying figures merely to facilitate an understanding of the various embodiments described herein and, accordingly, may not necessarily be presented or illustrated to scale, and are not intended to indicate any preference or requirement for an illustrated embodiment to the exclusion of embodiments described with reference thereto.

### DETAILED DESCRIPTION

Reference will now be made in detail to representative embodiments illustrated in the accompanying drawings. It should be understood that the following descriptions are not intended to limit the embodiments to one preferred embodiment. To the contrary, it is intended to cover alternatives, modifications, and equivalents as can be included within the scope of the described embodiments as defined by the appended claims.

Embodiments disclosed herein are directed to systems and methods for measuring respiratory function of a user. The system and methods include measuring respiratory parameters of a user while the user breathes at multiple different breathing rates. Changes in the user's chest movement between the multiple different breathing rates are used to determine one or more respiratory metrics for a user. For example, the system and methods can identify and/or characterize a level of breathing difficulty or airway obstruction of a user (cumulatively referred to herein as "respiratory obstruction" of a user).

The system and methods include measuring respiratory parameters of a user while the user performs a guided breathing exercise. The measurements taken during the guided breathing exercise are analyzed to determine a respiratory metric for the user. The system includes an electronic device that outputs instructions for performing the guided breathing exercise. The system also includes a sensor that measures movement of a user's torso during the guided breathing exercise. The guided breathing exercise includes instructing a user to breathe at a first breathing rate and measuring the user's torso movement at that first breathing rate. The guided breathing exercise also includes instructing a user to breathe at a second breathing rate that is different than the first breathing rate and measuring the user's torso movement at that second breathing rate. Changes in movement of the user's torso between the first and second breathing rates are used to determine one or more respiratory metrics for a user. For example, changes in torso movements between the two breathing rates can be used to determine whether a user has respiratory obstruction. As used herein the term "torso" is intended to include portions of the user's upper body that moves as part of the breathing motion of the user. Accordingly, the term torso can include, for example, a portion of the user's abdomen, chest, and/or back.

Spirometry is a typical procedure for diagnosing respiratory obstructions. During a spirometry measurement, an individual blows into a spirometry machine with one breath as hard and as fast as they can. The spirometer can measure a forced vital capacity (FVC), which is a measure of the largest amount of air an individual can exhale in a single breath; a forced expiratory volume (FEV), which is how much air an individual can force from their lungs in one second; and/or a peak expiratory flow (PEF), which is the maximum flow rate generated during the forceful exhalation. One or more of these measured parameters can be used to determine if an individual has a respiratory obstruction and/or a severity of the obstruction.

In some cases, spirometry measurements may be less accurate and/or less repeatable than desired due to forced exhalation procedure. For example, an individual may become better at the spirometry measurements over time, which may mask obstruction in the user. In some cases, individuals can have variability in the depth of their breath and/or the force of expiration between different measurements, which can affect accuracy and/or repeatability. Additionally or alternatively, maximum inhalation followed by a maximum force exhalation is not a typical breathing pattern for an individual, even at high exertion levels. Accordingly, spirometry measurements (e.g., FVC, FEV and PEF) may not be based on typical breathing patterns and/or indicative of respiratory function at a user's natural breathing rate.

In some cases, a user may not have regular access to a spirometry machine. For example, due to cost and/or size, spirometry machines may be located at medical facilities. Additionally, portable spirometry machines may not be as accurate as those located in a medical facility. Further an individual is more likely to deviate from the required protocols when not supervised by medical personnel. Accordingly, traditional spirometer measurements may not be as repeatable, accurate, portable and/or reflect typical breathing patterns of an individual as desired.

Embodiments described herein include a respiratory system that provides a guided breathing exercise to a user and measures respiratory parameters during one or more breathing measurement conditions. The system can include an electronic device that provides an audio, video, haptic, or other suitable output or combinations of outputs to a user. The outputs can include instructing a user to breathe at a first respiration rate, and the system can measure movement of the user's chest at the first respiration rate. In some cases, the first respiration rate is based on a natural respiration rate for the user (i.e., the rate at which a user will breathe when not prompted to breathe at a particular rate). For example, the system may monitor a user's natural respiration parameters while the user is in a resting state to determine the natural respiration rate, and the first instructed respiration rate can be determined from the user's natural respiration rate. The first respiration rate can be selected to be the same, greater than, or less than the user's natural respiration rate. The measurements may be taken over multiple respiration cycles. Measuring respiratory parameters at a user's natural respiration rate and/or over multiple respiration cycles may help increase measurement accuracy and/or repeatability. The outputs can also include instructing the user to breathe at a second respiration rate, and the system can measure movement of the user's torso at the second respiration rate over multiple respiration cycles. The second respiration rate may be different than the natural and first respiration rate. The second rate may be higher or lower form the first respiration rate; however, for the sake of simplicity, examples presented herein are presented in the context of the second breathing rate being greater than the first breathing rate, which is not meant to be limiting.

In some cases, the respiratory system can provide guided breathing based on one or more environmental conditions of the user. For example, the guided breathing protocol and/or when a guided breathing session is performed can take into account factors such as air quality, temperature, or other environmental parameters; user parameters such as heart rate, breathing rate, stress; timing and/or location parameters (e.g., based on GPS data); or other suitable parameters.

The system may determine a first respiration parameter for the first breathing rate and a second respiration parameter for the second breathing rate. The first and second respiration parameters can be used to determine a respiratory metric, for example, whether a user has a respiratory obstruction and/or a severity of the obstruction. In some cases, the first and second breathing parameters can indicate a user's ability to inhale and exhale at each of the requested breathing rates. For example, the system can include a sensor that measures changes in chest depth and the first and second parameters can be a breathing power, which may be based on the amount of chest movement over each of the sampling periods. In cases where the second breathing rate is greater, a decrease in the breathing power between the first breathing rate and the second breathing rate can be used to identify respiratory obstruction. For example, at the higher second breathing rate, an obstructed user may have a significant drop in their ability to inhale or exhale, which can be indicated by a drop in their breathing power. Breathing power is just one example respiratory parameter that can be determined and other parameters can be used in addition to or as an alternative to breathing power, some of which may include peak-to-peak amplitude, morphology of the measured chest movement, changes in morphology of measured waveform(s) (e.g., corresponding to changes in inhalation and/or exhalation portions of a respiratory cycle) and/or the like as described herein.

The system includes an electronic device that outputs instructions for the guided breathing exercise and includes a sensor for measuring torso movement of a user. In some cases, the depth sensor may generate a depth map including these calculated distances, some or all of which may be used in the various techniques described below. The depth information may be calculated in any suitable manner. In one non-limiting example, a depth sensor may utilize stereo imaging, in which two images are taken from different positions, and the distance (disparity) between corresponding pixels in the two images may be used to calculate depth information. In another example, a depth sensor may utilize structured light imaging, whereby the depth sensor may image a scene while projecting a known pattern (typically using infrared illumination) toward the scene, and then may look at how the pattern is distorted by the scene to calculate depth information. In still another example, a depth sensor may utilize time of flight sensing, which calculates depth based on the amount of time it takes for light (typically infrared) emitted from the depth sensor to return from the scene. A time-of-flight depth sensor may utilize direct time of flight or indirect time of flight, and may illuminate the entire field of coverage 118 at one time, or may only illuminate a subset of the field of coverage 118 at a given time (e.g., via one or more spots, stripes, or other patterns that may either be fixed or may be scanned across the field of coverage 118). Additionally or alternatively, optical flow processing of videos (e.g., RGB videos) can be used to extract respiratory metrics.

The electronic device can include a display, speakers, one or more microphones, and haptic output devices one or more of which may be used to present the guided breathing exercise to the user. In some embodiments the electronic device includes an optical sensing unit that can measure torso movements of the user. For example, the optical sensing unit can include a depth sensor that measures changes in depth of the torso of the user. These depth measurements can be used to determine respiratory parameters such as breathing power over one or more respiration cycles. Example electronic devices can include smartphones, tablets, smartwatches, or any other suitable electronic devices. In some cases, a first electronic device can output instructions for a guided breathing exercise and a second electronic device can measure chest movements of the user.

Additionally or alternatively, the system can include a motion tracking sensor that measures torso movements of a user. For example, the motion tracking sensor can be part of an electronic device and the electronic device can be placed on or otherwise coupled to a torso of a user (for the purpose of this application, an object is considered to be "coupled" to a torso of the user while it is held in a fixed relationship to the torso). The electronic device may be held in place relative to the torso of a user during a measurement such that the electronic device moves in unison with the chest portion. In some instances, a user may hold the electronic device in place against the chest (e.g., using their hand, or by placing their wrist against their chest while wearing a smartwatch). In other instances, the electronic device may be temporarily affixed to a user's torso (e.g., using a strap, fastener, adhesive, or the like). Accordingly, the electronic device may move with the user's torso and measure these torso motions. In some cases, the motion tracking sensor can include one or more accelerometers, gyrometers, wireless positioning systems, or other suitable sensors. The chest movement measured by the one or more motion tracking sensors can be used to determine respiratory parameters for the user, which can include breathing power, depth signal morphology, or other suitable parameters such as those described herein.

In some cases, the system can utilize multiple electronic devices to measure respiratory parameters for a user. For example, the system can include a first electronic device that has an optical sensing unit for measuring chest movement as described herein. The system can also include a second electronic device that includes a motion tracking sensor. For example, the first electronic device can be a smartphone or a tablet and the second electronic device can be a smartwatch. In some cases, wireless positioning can be used to track motion of a user's chest. For example, each of the electronic devices can include one or more antennas, and wireless signals transmission (e.g., radio-frequency, ultra-wide band signals, and so on) can be used to determine distances and/or orientation of the electronic devices with respect to each other. A first electronic device can be positioned at a distance from the user and the second electronic device can be coupled to the chest of the user, and the changes in distance between the devices can be used to determine chest movement of the user. This wireless positioning data may be used in addition to or as alternative to optical depth measurements, motion tracking, or other suitable processes. Additionally or alternatively, the system may use imaging and/or depth sensing functionality to identify and/or measure a distance between the first and second electronic devices.

Embodiments can include performing an enrollment period to determine one or more baseline data sets for a user, which may be used to identify respiratory conditions such as respiratory obstruction. Enrollment processes can include analyzing measurement conditions to determine whether suitable measurements can be made. For example, in some cases the clothing worn by a user may prevent the system from being able to detect sufficient torso movement. The enrollment process can include analyzing a user's clothing to determine whether the sensing systems can accurately measure respiration parameters, such as torso movements of the user. In some cases, the enrollment period can include measuring a user's normal breathing patterns and/or requesting a user to breathe at different breathing rates and measuring respiration parameters at the requested breathing rates. In some cases, the enrollment period can include measuring breathing parameters using traditional analysis methods such as spirometry measurements. The spirometry data can be compared and/or correlated to measurements taken by the respiratory sensing system described herein. Additionally or alternatively, the enrollment period can include analyzing a user's breathing parameters at different conditions such as standing positions, sitting positions, postures, mouth shapes, monitoring breathing sounds and so on.

The data obtained during the enrollment period can be used to generate one or more user models, which may be used to identify respiratory conditions such as whether a user is obstructed and/or a severity of respiratory obstruction. In some cases, the user model can be a parameterized model and enrollment data and/or data from respiratory sensing sessions can be used to generate a parameterized torso and/or body model. The parameterized model can include data related to a user's shape, pose, body composition, height, weight, and/or other demographic data. In some cases, the parameterized model can be generated using one or more images of a user, which may include images from various perspectives such as front, side, and/or back images of a user.

Respiratory measurements can be initiated in a variety of ways. In some cases, the respiratory measurements can be user activated, for example, by an application running on an electronic device. In other embodiments, respiratory measurements can be initiated based on a set schedule, which can be tracked by a calendar application, or other suitable process. In some embodiments, respiratory measurements can be initiated based on monitoring one or more physiological parameters of a user including heart rate, oxygen saturation, cardiac parameters (electrocardiogram (ECG) measurements), temperature, breathing sounds, and/or the like. In some cases, the system may evaluate one or more user parameters prior to initiating respiratory measurements, which can include determining whether suitable respiration measurements can be achieved (e.g., signal strength, suitable measurement region, and/or the like), evaluating a user's clothing, posture, and so on. Respiratory measurements can include at least one guided breathing session, and optionally an enrollment session. An enrollment session may be used to determine one or more parameters to help calibrate measurements taken during the respiratory measurements. An enrollment session may be performed for a first set of respiratory measurements, and in some instances may occur periodically thereafter, for example, to recalibrate measurements.

The respiratory measurements can include monitoring one or more user parameters and providing feedback to a user and/or adjusting the measurement conditions based on the measured parameters. For example, the system may instruct a user to breathe at a first breathing rate and monitor the user's actual breathing rate. The system may output an indication of the user's actual breathing rate and/or provide instructions to the user, which can include instructions to breathe faster, slower, more deeply and/or the like. In some cases, the system can determine the respiratory parameter based on the measured breathing parameters (e.g., breathing rate). The system can adjust the instructed breathing rate based on the measured parameters. For example, if a user is unable to meet the instructed rate, the system may decrease the instructed breathing rate. In some variations, the system adjusts the instructed rate when a measured breathing rate is below a predetermined threshold rate, which may be the instructed breathing rate or another breathing rate that is less than the instructed breathing rate. Accordingly, the system may actively adapt based on one or more conditions during a guided breathing session.

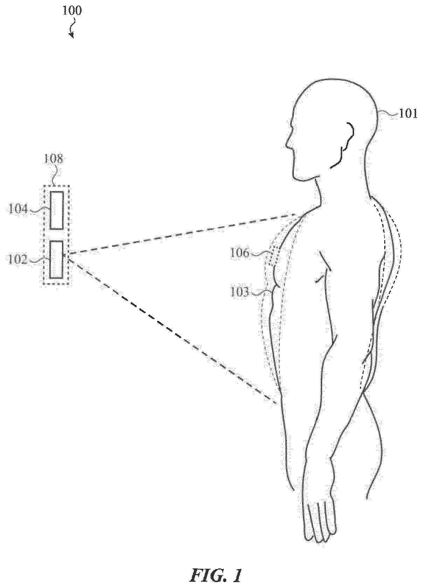

These and other embodiments are discussed below with reference to FIGs. 1-12. However, those skilled in the art will readily appreciate that the detailed description given herein with respect to these Figures is for explanatory purposes only and should not be construed as limiting.

FIG. 1 shows an example system 100 for determining respiratory function of the user 101. The system 100 includes a sensing unit 102 that is configured to measure one or more parameters of the user 101 and an output unit 104 that is configured to provide outputs to the user 101. In some embodiments that system 100 can include a second device 106 that contacts the user's 101 torso 103 and is configured to measure parameters of the user 101. The sensing unit 102, the output unit 104 and the second device 106 can be communicably coupled and may be operated in coordination to determine parameters of the user 101, such as one or more respiration parameters as described herein.

In some cases, the sensing unit 102 and the output unit 104 can be integrated into an electronic device 108 such as a smartphone, tablet, digital media player (e.g., mp3 player), smartwatch, laptop computer, desktop computer or other electronic device. The electronic device 108 may include a housing and a transparent cover (which may be referred to simply as a "cover") coupled with the housing and positioned over a display. The cover and the housing along with other components may form a sealed internal volume of the electronic device, which may contain the internal electrical components of the electronic device. In some cases, the cover defines substantially the entire front face and/or front surface of the electronic device 108. The cover may also define an input surface. For example, as described herein, the electronic device 108 may include touch and/or force sensors that detect inputs applied to the cover. The cover may be formed from or include glass, sapphire, a polymer, a dielectric, or any other suitable material.

The output unit 104 can include a display that is positioned under the cover and at least partially within the housing. The display may define an output region in which graphical outputs are displayed. Graphical outputs may include graphical user interfaces, user interface elements (e.g., buttons, sliders, etc.), text, lists, photographs, videos, or the like. The display may include a liquid-crystal display (LCD), an organic light emitting diode display (OLED), or any other suitable components or display technology. In some cases, the display may output a graphical user interface with one or more graphical objects that display information.

The display may be touch- and/or force-sensitive and include or be associated with touch sensors and/or force sensors that extend along the output region of the display and which may use any suitable sensing elements and/or sensing techniques. Using touch sensors, the electronic device 108 may detect touch inputs applied to the cover, including detecting locations of touch inputs, motions of touch inputs (e.g., the speed, direction, or other parameters of a gesture applied to the cover), or the like. Using force sensors, the electronic device 108 may detect amounts or magnitudes of force associated with touch events applied to the cover. The touch and/or force sensors may detect various types of user inputs to control or modify the operation of the device, including taps, swipes, multiple finger inputs, single- or multiple-finger touch gestures, presses, and the like.

Additionally or alternatively, the output unit 104 can include one or more speakers, which can be integrated with the housing of the electronic device 108. The speakers can be configured to provide audio outputs to the user 101, which can include instructions for guided breathing exercises, other user feedback and so on as described herein. The speakers can be part of the electronic device and/or integrated with other devices that are separated from the electronic device. For example, the output unit 104 may include one or more earbuds or headphones that are worn by the user and communicatively coupled with the electronic device.

In some cases, the output unit 104 can also include one or more haptic actuators that provide tactile outputs to the user 101. The haptic actuators can be part of the electronic device 108 and/or integrated on devices that are separate from the electronic device. For example, the electronic device 108 can include a smartphone that has an optical sensing unit 102 and the output unit104 can include one or more haptic actuators that are integrated with a wearable device such as a smartwatch. In this regard, the output unit 104 can include different components that are integrated with different devices and communicably coupled to provide coordinated outputs. For example, the display on the electronic device 108 may provide a visual cue for a guided breathing exercise, the speakers (integrated with the electronic device or other device such as headphones) can provide an audio cue that is coordinated with the visual cue and/or the haptic actuators (e.g., located on a smartwatch) may provide a haptic cue that is coordinated with the other outputs.

In some cases, the output unit 104 can be associated with a first electronic device and the sensing unit 102 can be associated with a different electronic device. The output unit 104 and the sensing unit 102 can coordinate respiratory measurements, which can include transmitting one or more signals between the sensing unit 102 and the output unit 104. In cases where the sensing unit 102 and the output unit 104 are associated with different electronic devices, signals can be transmitted between the different devices via a suitable wireless transmission protocol. The signals can indicate when the output unit 104 is outputting a first request for a user to breathe at a first rate. Additionally or alternatively, the signals can include a time period, duration and/or end associated with the first request. Accordingly, the sensing unit 102 can use these signals to associate measurement data with the first requested breathing rate and/or a time period associated with the first breathing rate. Additionally or alternatively, the signals can indicate when the output unit outputs a second or additional request for a user to breathe a different breathing rate. These signals can be used by the sensing unit 102 to associate measurement data with the second and/or additional requested breathing rates.

The sensing unit 102 includes an optical sensing unit that measures movement of the user 101. The optical sensing unit can include a depth measurement sensor (or sensors) that can determine a distance between the user 101 and the sensing unit 102. A depth measurement sensor may include a time-of-flight sensor, a structured light sensor, a stereo camera, or the like. The optical sensing unit 102 can include a camera or other suitable imaging device that is configured to capture an image of a scene (which may in turn be used to identify one or more regions on a user). For example, the optical sensing unit 102 can include a camera that can image the user's body and the optical sensing unit 102 and/or other components of the electronic device 108 (e.g., processor) can be configured to identify anatomical features of the user, such as a torso of a user. The depth sensor and the camera can have overlapping fields of view, such that the identification of anatomical features via the camera can be used by the device to associate anatomical features with measurements made by the depth sensor.

The electronic device 108 can include additional sensors such as accelerometers, gyrometers, positioning sensors such as global position system (GPS) sensors, wireless positioning systems, altimeters, pressure sensing systems and/or the like. Additionally or alternatively, the electronic device 108 can include physiological sensors such as temperature sensors, heart rate monitors and/or other suitable sensors.

The device 106 can be a wearable electronic device such as a smartphone or other suitable device. In some cases, the device 106 can contact the user's 101 chest by the user holding the device 106 against their chest. In other cases, the device 106 can be coupled to the user's chest, for example by a coupling device. Accordingly, the device 106 may move with the user's 101 chest as they breathe or perform other actions. The device 106 can be communicably coupled to the electronic device 108. The system 100 can measure movement of the user's 101 chest by tracking movement of the second device. For example, the electronic device 108 and the device 106 can each include one or more antennas. The electronic device 108 and the device 106 can transmit signals, such as UWB signals, that can be used to determine distance and/or positions of the device with respect to each other. Accordingly, the electronic device 108 and the device 106 can track movement such as changes in depth of the user's 101 chest using wireless based distance and position sensing. Additionally or alternatively, the device 106 can measure movement of the user's 101 chest based on one or more onboard sensors such as accelerometers, gyrometers, or other suitable position sensors. Additionally or alternatively, a camera, distance sensor or other sensor from the sensing unit 102 can be used to identify the device 106 and/or track movement of the device 106 while it is positioned on the user's 101 torso 103. In some cases, the device 106 may include a display that displays a predetermined image which can be used by the sensing unit 102 to identify and/or track the device 106.

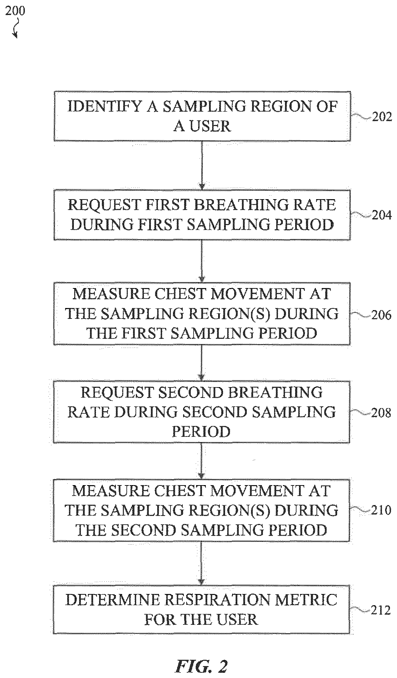

FIG. 2 shows an example process 200 for determining respiratory function of a user. The process can be performed by the respiratory sensing systems described herein such as a system 100.

At 202, the process 200 can include identifying one or more sampling regions along a user for measuring respiratory parameters. The sampling regions can be located across a chest and/or abdomen of a user. In some cases, the sensing unit can include an optical sensing unit that has one or more cameras that image a torso of the user and identify anatomical features such as a profile of the user's torso, location and/or movement of the user's shoulders, and so on. The sampling regions can be defined based on the identified anatomical features. For example, the system can define multiple sampling regions at different locations along the user's chest and/or abdomen. In some cases, the system may select a sampling region based on the identified anatomical features. The system may measure movement at each of the sampling regions. For example, a camera may be used to identify anatomical features of the user, which are used to define one or more sampling regions. A depth sensor can measure changes in depth of the torso at each of the sampling regions. In some cases, a parameterized body model of the user can be fit to the measurement data to separate breathing induced movements from other movements.

In some cases, the system may select one or more of the sampling regions for generating respiratory parameters of a user. For example, the system may compare signal strength, accuracy, measurement stability, and/or the like at each sampling region and select one or more sampling regions based on these metrics. For example, the process 200 may include selecting the sampling region that has the greatest signal strength and/or range of depth measurements. The depth measurements at the selected sampling region(s) can be used to determine respiration parameters such as breathing power, as described herein.

Additionally or alternatively, the system may monitor and collect data for each defined sampling region during one or more sampling periods. The operation 202 can include normalizing the measured chest movement data for each sampling region. Accordingly, the system may be able to use measurement data from different sampling regions during one or more of the sampling periods. If the user moves, the optical sensing unit moves, or other changes occur that affect the signal quality at the different sampling regions, the operation 202 can include dynamically selecting a different sampling region or set of sampling regions for determining a respiration parameter. In some cases, the relative movement between the user and the optical sensing unit may be large enough where a new sampling region(s) is identified and a new selection of a sampling region can occur. Accordingly, a respiration parameter may be based on measurement data from different sampling regions.

At 204 the process 200 includes instructing the user to breathe at a first rate during a first sampling period. For example, an output device such as a display, speaker and/or haptic device can output instructions for a user to breathe at a first rate. An electronic device can use a display to provide visual outputs corresponding to a first breathing rate. The visual outputs can be implemented in a variety of ways including dynamic outputs that indicate inhale and exhale timing, which a user of the system can mimic to achieve the instructed breathing rate.

In some cases, the first breathing rate can be a constant breathing rate such as a defined number of breaths per time. In other cases, the first breathing rate can be dynamically selected based on one or more user parameters captured before a guided breathing session such as a user's natural breathing rate and/or one or more parameters captured during the guided breathing session such as their heart rate, their current breathing rate, and/or the like. For example, a first value for the first breathing rate can be selected if a user's heart rate (which may be determined using another sensor system) is below a threshold and a second value for the first breathing rate can be selected if the user's heart rate is above the threshold. In some cases, the first breathing rate can change during the first sampling period. For example, the first breathing rate can change according to a defined protocol such as a constant increase in the instructed breathing rate (e.g., ramp protocol) and/or increase followed by decreases in the instructed breathing rate (e.g., cyclic protocol). In any of these cases, the outputs can dynamically change to indicate the desired breathing rate and/or changes to the user. In some cases, the instructed breathing rate can be based on current breathing metrics for a user. For example, the system can measure a current breathing rate of the user, for example in response to the instructed breathing rate, and update the instructed breathing rate based on the user's currently measured breathing rate. This may be used to adjust the instructed first breathing rate, for example, in cases where the user is not able to match the requested breathing rate, it can be updated to a slower rate.

In some cases, adjusting the breathing rate may help facilitate measurement accuracy by obtaining data during the first sampling period that provides a baseline data of a user's inhalation and exhalation capacity. For example, the first breathing rate may be set to allow a user to fully inhale and fully exhale. Accordingly, in some cases, the first sampling period collects baseline data for a user that establishes respiratory parameters for the user at resting/ lower exertion conditions.

The system may set the duration of each sampling period to collect one or more respiration cycles. In some cases, the sampling period can be a defined period of time that captures measurement data at the first requested breathing rate for at least two or more full respiration cycles for the user. In other cases, the sampling period can be a defined number of breathing cycles, which may vary in time depending on the requested rate and/or the user's actual measured breathing rate. In other examples, the duration of the sampling period can be dynamically adjusted based on measured respiration data for the user. The system can analyze collected data during the sampling period, the results of which can be used to adjust the sampling period. For example, the system may analyze averages, deviations or other metrics associated with the measurement data that is being collected, and adjust the sampling period based on these metrics. For example, if movement of the sensing unit negatively impacts the quality of measurement data, the system may extend the sampling period. In some cases, this can include having the first sampling period run until a defined metric is satisfied, which may include sampling until a deviation between measured respiration cycles satisfies a threshold. In other cases, the system can analyze collected data during the sampling period.

At 206, the process 200 includes measuring movement of one or more sampling regions during the first sampling period. This can include measuring changes in the depth of the chest, which correspond to expansion and contraction of the chest due to respiration. The optical sensing unit can be part of an electronic device, such as a smartphone, and the electronic device can include additional sensors such as accelerometers, gyroscopes, GPS positioning systems, other wireless positioning systems, altimeters, and/or other position sensors. These sensors can monitor motion of the electronic device thereby determining whether the optical sensing unit moves during the first sampling period. In cases where the optical sensing unit is stationary, distance measurement by the optical sensing unit may directly correlate to depth changes of the sampling regions along the user's chest. In cases where the optical sensing unit is moving, measurement data from the position sensors (accelerometers, gyroscopes, etc.) may be used to account for movement of the optical sensing unit and determine depth changes of the sampling regions along the user's chest. Accordingly, the electronic device may be able to determine depth changes when the electronic device is stationary and/or if the electronic device moves or changes orientation during the sampling period.

Additionally or alternatively, the operation 206 can include measuring body movements of a user and compensating for those body movements to determine chest movement. During the sampling period, the user's body (e.g., entire torso) may move relative to the optical sensing unit due to the user changing position, shifting, or making some other gross body motion. The optical sensing unit may track these gross body motions to differentiate them from chest movements resulting from breathing. For example, a parameterized body model of a user can be used to differentiate and/or extract breathing induced changes for other measurement data such as gross body motion. In some cases, the optical sensing unit can use one or more cameras to identify anatomical features of the user, which can include identifying a profile of the user's torso, identifying anatomical features such as the user's shoulder, neck, head, abdomen and so on. In some cases, the optical sensing unit can measure distance changes of theses different anatomical regions. Accordingly, movement measurements from the one or more sampling regions can be compared to distance measurements of one or more of these anatomical regions, and chest (and/or abdomen) movements can be separated from other motions of the user.

In some cases, the sensing unit can include a position sensing unit that contacts and/or is coupled to one or more sampling regions on the user. The position sensing unit can measure movement of the torso by movement of the device. For example, the movement of the position sensing unit may be tracked using motion sensors (e.g., accelerometers, gyroscopes, and so on) or by measuring relative motion between the position sensing unit and another device, as described herein. The position sensing unit can be in addition to the optical sensing unit or used as an alternative sensing system. For example, the position sensing unit can include a smartwatch that is worn by the user and the user can be instructed to place their hand against their chest to measure chest movements via movements of the smartwatch. In other examples, the smartwatch or other position sensing device such as a low-energy near-field tracking device can be coupled to a chest region of the user. For example, a coupling device such as a band, strap, adhesive-based device, or other suitable device may be used to couple the position sensing device to a sampling region of the user. Accordingly, the motion of the position sensing device may correspond to chest movements of the user, which can be used to determine depth changes and/or other movements of the user during the first sampling period.

At 208, the process 200 includes instructing the user to breathe at a second rate during a second sampling period. This can be implemented similar to operation 204 and include different types of outputs such as visual, audio, and/or haptic as described herein. In some cases, the second breathing rate may be a faster breathing rate than the first breathing rate. In other cases, the second breathing rate may be slower or configured with different parameters from the breathing rate during the first sampling period. In some cases, the second sampling period can be performed during a same guided breathing session as the first sampling period. For example, the guided breathing session can include performing the first sampling period followed by the second sampling period. Accordingly, there may be multiple sampling periods during a continuous guided breathing session.

The second breathing rate may be a predefined breathing rate, for example a breathing rate that is a defined amount quicker than the first breathing rate, for example double the first breathing rate. In some cases, the second breathing rate may be selected using a predetermined relationship to the first breathing rate. For example, the second breathing rate could be a rate that produces a statistically predictable drop in breathing power for specific conditions. For example, the second breathing rate may be selected to produce a first expected drop in breathing power for non-obstructed individuals and a second expected drop in breathing power for obstructed individuals (as compared to breathing power at the first breathing rate).

In some cases, the second breathing rate can be specific to each user. For example, the second breathing rate can be determined from the user's baseline data determined during an enrollment process. The second breathing rate can be a defined increase from the user's natural breathing rate. The defined increase in the breathing rate may be based on empirical data that would produce a first result for non-obstructed users and a different result for obstructed users.

At 210, the process 200 includes measuring movement of one or more sampling regions during the second sampling period, which can be implemented similar to operation 206. This can include measuring changes in the depth of the chest, which correspond to expansion and contraction of the user's chest due to respiration as described herein.

At 212, the process 200 includes determining a respiration metric for the user. The respiration metric is based on the respiration parameters that were generated from the first and second sampling periods (and/or additional sampling periods). In some cases, the respiration parameters can include a breathing power of the user at each of the requested breathing rates. As described herein, the breathing power may correspond to what extent a user is able to inhale and exhale at each of the instructed breathing rates (i.e., the amount the chest moves during inhalation and exhalation). The breathing metric may be a categorical classification that is determined from the respiration parameters. For example, the breathing metric can categorize the user as to whether they are experiencing respiratory obstruction or not. In some cases, the user can be categorized based on a change between the first respiration parameter measured during the first sampling period and the second respiration parameter measured during the second sampling period (and/or additional sampling periods). For example, if the breathing power drops more than a defined threshold between the first sampling period and the second sampling period, operation 212 can categorize the user as experiencing a respiratory obstruction.

Additionally or alternatively, operation 212 can include determining a severity of obstruction. For example, data may indicate that a non-obstructed individual will fall within a first range of decreased breathing power, a mildly obstructed user will fall within a second range of decreased breathing power, a moderately obstructed user will fall within a third range of decreased breathing power, and so on. In this regard, the change (e.g., decrease) in a user's breathing power between the first respiration rate and the second respiration rate can be used to classify a severity of respiratory obstruction.

Two different sampling periods are described herein for the sake of illustration. Accordingly, additional sampling periods including additional breathing rates (increasing and/or decreasing) can be used in some cases. For example, a third sampling period can be performed in which a third set of chest movement measurements is obtained at a higher breathing rate than the first and the second sampling periods. The measurements at these additional relationships may be used to determine obstruction and/or a severity of obstruction. In some cases, the process 200 can include additional sampling periods that include different breathing rates/patterns and/or repeated breathing rates/ patterns from one or more of the first and second sampling periods. Additional sampling periods may be used to develop more robust breathing data at one or more rates, to improve analysis of the data (e.g., statistical significance) and/or to collect additional data at different conditions (e.g., different breathing rates/patterns from the first and second sampling periods).

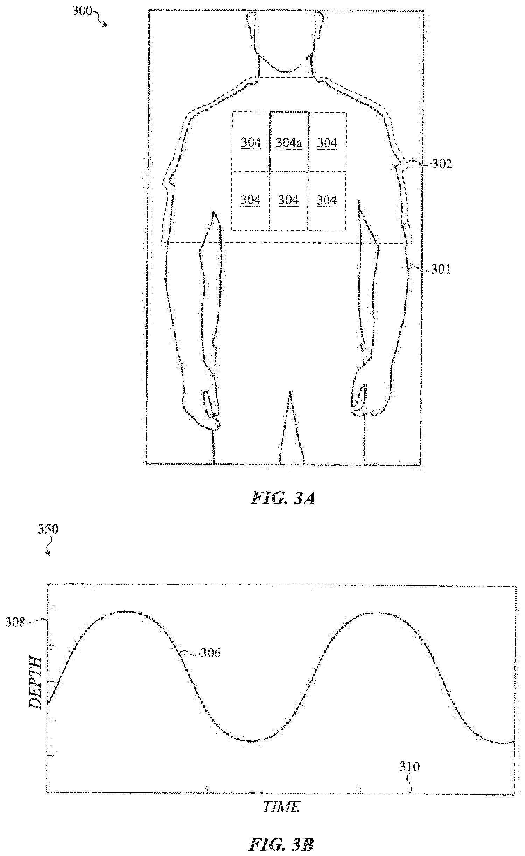

FIGs. 3A and 3B show an example chest movement analysis for determining respiratory parameters of a user 301. The example chest movement analysis is illustrated visually in FIGs. 3A and 3B, however, one or more of these processes can be performed by a computer and may not produce and/or display the graphical elements displayed in FIGs. 3A and 3B.

FIG. 3A shows an example of determining one or more sampling regions 304 that are determined from a field of view 300 of one or more of the sensing systems. The one or more sampling regions 304 can be locations for measuring chest movement of the user 301 during a sampling period. An optical sensing unit identifies a torso 302 of the user 301. In some cases, this can include using a camera to capture one or more images of the user 301, which can include capturing a real-time/continuous video feed of the user 301. In some cases, the images captured by the camera can be analyzed in real-time or near-real time to identify changes in the user 301 such as changes in position, posture, orientation, and so on. The analysis can be continually updated to account for these changes captured by the camera. The camera may be capable of capturing color images, black and white images, and/or non-visible (e.g., infrared) images, which may be used to provide information about the user 301 and the surrounding environment. The images captured by the camera can be analyzed to differentiate the user 301 from the surrounding environment. For example, the image analysis may include determining a profile or boundary of the user 301. The torso 302 can be based on the identified boundary and anatomical features of the user 301.

Identifying the torso 302 can be implemented in a variety of ways. In some cases, the torso 302 can include the user's 301 shoulders and a portion of the user's 301 torso such as an upper portion of the torso as illustrated in FIG. 3A. In other cases, the torso 302 can include a lower portion of the user's 301 torso such as an abdomen. The torso 302 may be identified based on image analysis that includes identifying specific anatomical features of a user and then defining the region of torso 302 based on these features. For example, the analysis can include identifying the upper boundary of the user's 301 shoulders and/or other prominent features such as an elbow or waist region of the user 301 and defining the torso 302 as the profile of the user 301 between the shoulder (e.g., upper boundary) and the elbow region (e.g., lower boundary). In some cases, the system can instruct the user to stand in a specific position or posture, and/or perform specific movements, which may be used to identify one or more anatomical features of the user 301.

Any suitable image analysis techniques may be used to identify the user. In some cases, information from an image (e.g., color information) can be used to differentiate between image data associated with the user 301 and image data associated with the user's surroundings. Additionally or alternatively, movement of the user 301 can be used to identify anatomical features of the user 301. For example, movement of the user 301 with respect to stationary background elements can be used to define a profile of the user 301 within the image data and/or determine anatomical features of a user.

The identified torso 302 can be used to define one or more sampling regions 304. For example, the sampling regions 304 can be an array of regions having a defined size that are positioned within the torso 302. One or more depth measurements can be taken within each sampling region and combined to produce measurement data for each of the sampling regions 304. In some cases, the depth measurements taken at each sampling region 304 can be averaged or otherwise combined to generate region data. The measurement data for each sampling region can be normalized, which can be based on the total depth changes within a region. The normalization may allow different regions to be compared, for example, because a central torso may have greater absolute movement than a peripheral chest region. For at least this reason, normalizing measurement data across different sampling regions may allow data for be collected for each region and data from different regions may be used to generate a respiration parameter. This region-based analysis may help provide robust analysis, during a sampling period, for example the region analysis may use data from different ones of the sampling regions 304 as the user 301 and/or the optical sensing unit moves and/or changes positions during the sampling period.

FIG. 3B shows an example of measurement data 350 collected from sampling region 304a during a sampling period. The measurement data 350 can include chest movement 306 which include depth changes over time. The measurement data 350 is shown as a graph that includes depth measurements 308 over time 310, for the sake of illustration. Although as implemented, this data may be process by a computer system, as described herein, and be stored and processed in computer readable formats such as binary data.

The measurement data 350 shows chest movement 306 for the sampling region 304a. In some cases, the measurement data 350 can include chest movement measurements for one or more of the other sampling regions 304. In some cases, the measurement data 350 can be analyzed in the frequency spectrum and one or more respiration parameters can be derived from the frequency-based analysis such as identifying a fundamental frequency using Fourier analysis.

In some cases, the measurement data 350 may not correspond to defined regions as illustrated in FIG. 3A. For example, the system may collect an array of data each corresponding to different locations across the torso 302. Each sampling point may be normalized and a depth map may be generated based on the combination of the sampling points. For example, each sensing location may correspond to a time-based depth signal. In some cases, this data may be used to determine how breathing movement propagates across the user's 301 torso 302. For example, movement propagation can be used to determine whether breathing movement is initiated in the upper chest region (e.g., chest area corresponding to the lungs) and/or whether and/or to what extent the user 301 is using their abdomen region during respiration. In some cases, different portions of a depth map can be compared to provide an indication of to what extent a user is using their chest versus their abdomen. In other cases, one or more sampling regions corresponding to a chest region of a user may be compared to one or more sampling regions corresponding to an abdomen of a user to determine to what extent a user is using their chest region versus their abdomen. Analyzing chest versus abdomen movement may be informative of respiratory function such as whether the user 301 is experiencing obstruction.

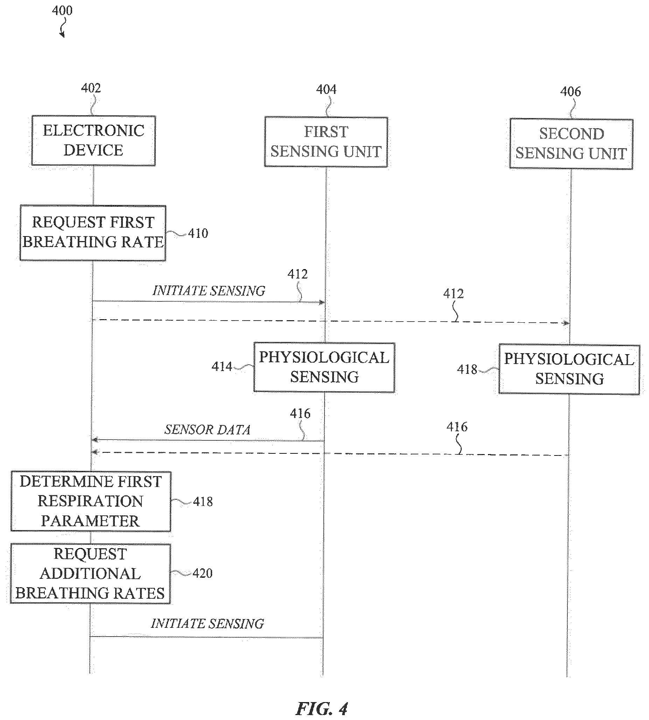

FIG. 4 shows an example signal flow 400 between different devices of a respiratory monitoring system, such as the respiratory monitoring systems described herein. The signal flow 400 can be between an electronic device 402, a first sensing unit 404, and a second sensing unit 406 that measure chest movement of a user. The electronic device 402 may be an example of the electronic devices described herein that have an output unit that outputs instructions for a user to perform guided breathing exercises as described herein. In some cases, the electronic device 402 can include the first sensing unit 404 or the second sensing unit 406. For example, the electronic device 402 can be a smartphone and include an optical sensing unit as described herein. In some cases, the second sensing unit 406 can be integrated into an electronic device that contacts a user and measures chest movements of the user as described herein. The electronic device 402 may correspond to device 108 and output unit 104 of the system described herein and with respect to FIG. 1. The first sensing unit 404 may correspond to the optical sensing unit 102 and the second sensing unit 406 may correspond to a second sensing unit such as a sensing unit of the electronic device as described herein and with respect to FIG. 1.

At 410, the electronic device 402 outputs a request for a user to breathe at a first breathing rate as described herein. In response to outputting the request, the electronic device 402 can initiate a respiration measurement process at one or more of the first sensing unit 404 and the second sensing unit 406. The respiration measurement process includes performing optical sensing of chest movements of a user. At 412, the electronic device may send one or more signals that initiate an optical sensing process at the first sensing unit 404. For example, the first sensing unit can identify a torso of a user and detect depth changes of the user's chest. In other cases, the respiration process can include performing a motion measurement process using the second sensing unit that is contacting the user. In these cases, at 412, the electronic device 402 may transmit one or more signals to the second sensing unit 406 to initiate a motion sensing process.

In some cases, the first sensing unit 404 and the second sensing unit 406 may operate together to perform respiration measurements for a user. For example, the fist sensing unit 404 may track movement of the second sensing unit 406, which may correspond to movement of the user's chest. In some embodiments, the first and second sensing units 404, 406 can use wireless position sensing to determine chest movement. For example, each of the first and second sensing units 404, 406 can include one or more antennas and may transmit wireless signals (e.g., ultrawideband signals) between various ones of the antennas, which can be used to determine distance and/or positioning of the devices with respect to each other. This positioning data can be used to determine movement of a user's chest, such as changes in depth due to movement of the second sensing unit 406 with respect to the first sensing unit 404.

In other embodiments, the first sensing unit 404 can use the second sensing unit 406 as a visual target and can optically track the second sensing unit 406 to measure chest movement of a user. The second sensing unit can be an electronic device as described herein. In other cases, the second sensing unit 406 can be a device that does not include electronic components, but instead functions as an optical target for the first sensing unit 404. For example, the second sensing unit can be a device that couples to a chest of a user and provides an optical target for the first sensing unit 404.

In response to the electronic device sensing one or more signals to initiate physiological sensing at 412, the first sensing unit 404 can measure physiological parameters of a user at 414 and/or the second sensing unit 406 can measure physiological parameters of a user at 418. In cases where the first sensing unit 404 comprises an optical sensor, at 414, the first sensing unit may generate signals indicative of depth changes of the user's chest over a sampling period. Additionally or alternatively, the second sensing unit 406 may measure motion of user's chest using one or more motion sensors as described herein and generate signals indicative of the measured motion. The motion signals can include accelerometer outputs, gyroscope outputs, and/or other position sensing data.

In cases, where the first and second sensing units 404, 406 operate in coordination to track chest movements, the devices containing these sensing units may establish one or more communication channels that can be used to coordinate sensing activities and/or exchange sensing data. For example, when wireless signal positioning is used, the first sensing unit 404 may include one or more antennas that transmit wireless signals to the second sensing unit 406. The second sensing unit may include one or more antennas the receive the wireless signals, which can be any suitable radio-frequency signals including ultra-wide band (UWB) signals. Distances between different sets of antennas in the first and second sensing units 404, 406 can be determined based of signal transmission time, and the distance and/or position between the first and second sensing units 404, 406 can be determined from these signal transmissions. One or more of the electronic devices associated with the first and second sensing units 404 and 406 can determine depth changes based on the measuring distance and/or position changes between the sensing units.

At 416, the first sensing unit 404 and/or the second sensing unit 406 can transmit the measurement data to the electronic device 402. In some cases, the measurement data may include digitized signals that are indicative of the measured parameters and the electronic device 402 can process these signals to derive one or more respiration parameters for the sampling period. The signals can include time stamps or other data that can be used to correlate signals received from the first sensing unit 404 with the signals received from the second sensing unit 406. Accordingly, the measurement data from each of the sensing units can be compared and/or combined to determine respiration parameters for the user.

At 418, the electronic device can determine a first respiration parameter for the user based on the measurement data received from the first and second sensing units 404 and 406. In some cases, the electronic device 402 may determine a respiration parameter using data from the first sensing unit 404 and independently determine a respiration parameter using data from the second sensing unit 406. The electronic device 402 may compare the two respiration parameters to generate a combined parameter. In other cases, one of the sensing units may be operated to perform a primary measurement and the other sensing unit can be operated to perform a second measurement that is used to supplement or update the primary measurement. For example, the first sensing unit 404 can be operated to measure changes in chest depth of the user and the second sensing unit 406 can be operated to measure sources of noise such as gross movement of the user, transient chest movements such as due to coughing, and so on. In some cases, the second sensing unit 406 can measure secondary parameters such as wheezing, breathing sounds, and so on which can be analyzed with the primary measurements to determine one or more respiration metrics for the user.

At 420 the electronic device can determine whether to perform additional respiratory measurements. For example, the electronic device may output a request for a user to breathe at a second rate, and in response, can initiate a second sensing procedure at the first sensing unit 404 and the second sensing unit 406, which can be similar or the same as described with respect to the first sampling period at the first requested breathing rate.

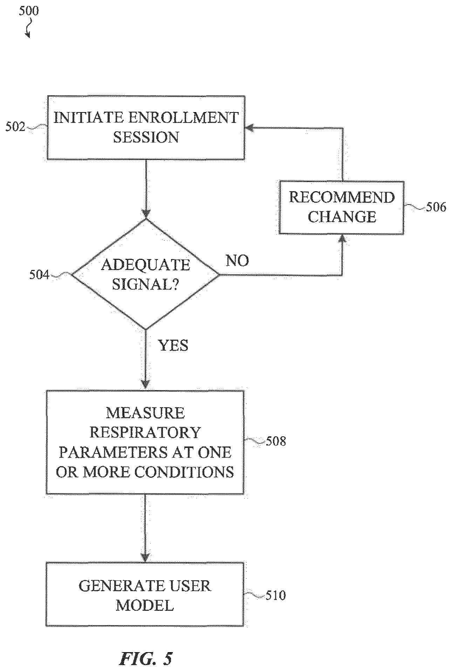

FIG. 5 shows an example process 500 for performing an enrollment session for respiratory sensing. The process can be performed by the respiratory sensing systems described herein such as a system 100. During the enrollment session, the system can collect and analyze data about the user that can be used as a baseline data set for the user. After the initial enrollment period, data collected by the system can be compared to the baseline data set to predict and/or determine whether a user is experiencing a health episode such as respiratory obstruction. The baseline data can include both data associated with stable conditions such as when a user is not experiencing health condition symptoms, as well as data associated with the user experiencing symptoms of one or more health conditions such as a respiratory obstruction.

At 502, the process 500 can include initiating an enrollment session for a user of the respiratory sensing system. In some cases, the enrollment session can be activated the first time that a user initiates respiratory sensing function of the electronic device. For example, the enrollment session may be activated in response to a user opening an application for respiratory sensing. In some cases, initiating an enrollment session can include collecting data about a user. The system may collect physiological parameters for a user. The enrollment session can be performed for a defined amount of time or until stable baseline data sets are developed for the user. For example, determining a stable baseline data set can include determining values or ranges for specific physiological parameters within a defined confidence interval. After the enrollment session has ended, current sensor data can be compared to the baseline data sets to determine whether a user is exhibiting symptoms indicative of a respiratory condition.

In some cases, the enrollment session include measuring respiratory parameters using traditional respiratory measurement techniques including spirometry measurements, which can be compared to measured chest movement data obtained by the systems described herein, used to calibrate chest movement measurement by the systems described herein, and/or otherwise used in the respiratory measurement analysis. In some cases, the system (e.g., the output unit or other electronic device) can perform guided breathing for spirometry measurements or other forced exhalation techniques. For example, the electronic device can output video, audio, haptic cues to guide the user through a forced exhalation protocol such as a spirometry measurement.

At 504, the process 500 can include determining whether adequate respiratory measurements can be achieved. For example, the ability to get an adequate signal strength, signal quality and/or repeatability over a sampling period may be dependent on a variety of environmental factors. These can include the positioning of the sensing unit(s) with respect to the user, movement of the sensing unit(s) and/or the user, background noise, and so on. In cases where the sensing unit includes an optical sensor, lighting, the clothing being worn be the user, user and/or camera movement, and/or the like can all affect the signal strength and/or quality. Accordingly, at 504 the process 500 may evaluate one or more conditions to determine whether adequate respiratory measurements can be achieved.

In some cases, the system can evaluate the positioning of the sensing unit(s) with respect to the user, which may include determining whether a torso of a user is within a field of view of an optical sensor, the signal strength of one or more sensors, signal noise, movement of the sensors and/or user, and so on. In cases where an optical sensing unit is being used, the system may analyze a user's clothing to determine whether it can measure a user's chest movement. For example, if a user's clothing is sized or positioned to not be in contact with the user's torso, the system may not be able to detect chest movement of the user through the clothing.

At 506, the process 500 can include recommending changes to the user to improve the respiratory measurements by the system. For example, in the cases where a user's clothing sufficiently masks chest movement of the user from optical depth measurements, the system may prompt the user to adjust or change clothing or use a different sensing modality such as a movement sensor that is coupled to the user's chest. In other cases, the system may instruct the user to change the positioning of a sensing unit, such as an electronic device that has a camera, and/or instruct the user to change position and/or posture. In some cases, the system can provide live feedback on a display or using other outputs (e.g., audio instructions) for the user, for example, where to move the electronic device or how to reposition themselves within a field of view of the camera.

At 508, the process can include measuring respiratory parameters of a user to generate a baseline data set for the user. This can include measuring respiratory parameters at one or more different conditions. For example, the system may measure a user's natural breathing patterns by instructing the user to position themselves in front on the optical sensing unit and breathe naturally for a period of time. The system may perform multiple of these natural breathing sessions to develop a resting or natural breathing profile for the user. Additionally or alternatively, the system may instruct the user to breathe at one or more faster or slower breathing rates. The system can measure physiological parameters of the user at each of the different breathing rates, which can include measuring chest movement, heart rate, the user's actual breathing rate, oxygen saturation, temperature, breathing sounds and/or other physiological parameters. In some cases, the system may evaluate a user's ability to match a requested breathing rate, whether a user is within a defined range of the requested rate, consistently faster, consistently slower, and so on.

In some cases, the during the enrollment session, the system can correlate measurements from the sensing unit(s) to other measurement modalities. For example, the process 500 can include taking spirometry measurements during the enrollment session. The spirometry measurement data can be correlated to data measured by the respiratory sensing system, which can include correlating FVC, FEV and PEF to the chest measurement data from the respiratory sensing system. In some cases, chest motion data measured by a first sensing modality, such as optical depth sensing, can be correlated with physiological data measured by a second sensing device such as a wearable health monitoring device. For example, the second sensing device can include a smartwatch that measures heart rate, ECG data, oxygen saturation, and/or the like.

Additionally or alternatively, the system can analyze the effect of different user positions/postures on the user's breathing motion. For example, the system may instruct the user to perform different breathing sessions in different positions such as sitting, standing, laying down, from a front view, from a back view, and/or at different postures such as standing upright versus hunched over. This data may be integrated into the baseline data set such that the user can collect respiratory motion data in a variety of different positions/postures and the baseline data can be used to normalize these differences. In other cases, the system may instruct the user to perform a breathing exercise in a specific posture to increase the accuracy, repeatability and/or to otherwise improve the breathing measurements.

In some cases, the system may instruct the user to breathe in particular manner during a given breathing session. For example, it may be desirable for a user to breath in a consistent manner across breathing sessions. Accordingly, the system may select a particular target breathing style (i.e., nose breathing vs mouth breathing) and/or target mouth condition (e.g., mouth opened, mouth closed, mouth opened with a particular shape), and may provide feedback to the user depending on whether the user is sufficiently meeting the target breathing style and/or target mouth condition. For example, the system may instruct the user to breathe with their mouth open. In other cases, the system may instruct the user to breath through their nose and with their mouth closed. The system may use information from the cameras to determine a user's mouth shape and/or an extent that they are breathing from their mouth or nose. In some cases, the system may provide feedback to the user to guide breathing through their mouth or nose. For example, the system may provide feedback requesting a user to open their mouth wider, close their mouth and breathe through their nose, or breathe at other mouth/nose conditions.

At 510, the process 500 can include generating a user model (e.g., parameterized model) based on the data collected during the enrollment session. The user model may be part of the baseline data set and include three-dimensional model data for a user. For example, the system may generate three-dimensional model data for a user's torso which can be based on sensor data and/or data input from a user. The sensor data and/or user inputs can include parameters related to a user's height, weight, sex, and/or the like. In some cases, this data can be used to derive and/or generate model data such as a body mass index, a body surface area, dimensions/proportions of a user's torso, lung capacity, and/or the like. The user model can be used to refine the respiratory analysis, for example to help determine how user specific factors such as the user's body shape, lung capacity and so on affect a user's breathing movements. In some cases, the user model may help increase the accuracy of determining whether a specific user is experiencing respiratory obstruction and/or a severity of the obstruction.

In some cases, the user model can be updated over time. For example, as respiratory sensing sessions are performed, data from these sessions can be used to update and/or track changes in the user model. In this regard, the system may track long-term changes for a user and/or adapt to changing respiratory conditions of a user. In some cases, this long-term data may provide data that can be used to diagnose improving, worsening, or stable respiratory conditions over longer periods.

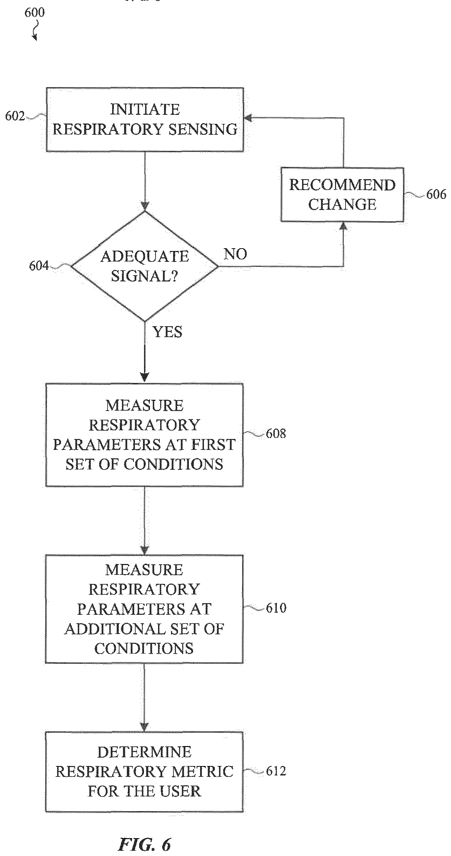

FIG. 6 shows an example process 600 for performing a respiratory sensing session for a user. The process can be performed by the respiratory sensing systems described herein such as a system 100.

At 602, the process 600 can include initiating a respiratory sensing session. A respiratory sensing session can be initiated in a variety of ways. In some cases, the respiratory sensing session can be initiated by a user. For example, an electronic device can include an application for performing respiratory measurements and the application can include controls for a user to initiate a sensing session. In other cases, the sensing session can be initiated in response to one or more conditions. For example, the sensing session can be performed on a defined schedule. In other embodiments, the sensing session can be performed in response to a specific event such as an event managed by a calendar application. For example, a sensing session can be initiated prior to a scheduled exercise session, after an exercise session, and/or other calendar events scheduled for a user.

In some cases, a sensing session can be initiated based on a physiological parameter of a user. For example, a wearable or other device may track one or more physiological parameters of a user including a user's respiratory rate, respiratory sounds, heart rate, oxygen saturation, ECG data, temperature, movement parameters, and so on. The sensing system may receive this data and initiate a respiratory sensing session in response to one or more of these physiological parameters satisfying a criterion. For example, the system may monitor a user's breathing sounds and initiate a respiratory sensing session in response to the breathing sounds exceeding a sound threshold and/or indicating that the user is experiencing abnormal breathing.

At 604, the process 600 can include determining whether an adequate respiratory measurement can be achieved, which can be the same or similar to operation 504 of process 500. Evaluating the signal strength and/or quality can be performed prior to each sensing session and/or during the sensing session. In some cases, this can include evaluating a user's clothing, posture, and positioning with respect to the sensing unit(s), and/or the like as described herein. If at 604, the system determines that an adequate signal cannot be achieved the system may make one or more recommendations to the user at 606 to help improve the measurement signals, which can be similar to operation 506 of process 500. For example, the system can instruct the user to change clothes, move to reposition themselves within a field of view of a sensing unit, move the electronic device containing the sensing unit and so on.

At 608, the process 600 includes measuring respiratory parameters of the user at a first set of conditions. As described herein, measuring at the first set of conditions includes instructing a user to breathe at a first rate during a first sampling period. The system can define one or more sampling regions along a torso of a user. The system can use a sensing unit such as an optical sensing unit to measure changes in depth along the one or more regions. In some cases, the system can select at least one sampling region and determine a respiratory parameter using the measured depth changes at the selected sampling region(s).

In some cases, the first respiratory parameter can include a breathing power for the sampling period, which may be based on the total chest movement of the user. The breathing power may provide an indication of to what extent the user inhaled and exhaled at the first breathing rate. The respiratory sensing system may determine one or more additional or alternative breathing parameters based on the measured depth changes during the first sampling period which can include determining peak-to-peak amplitude of the user's chest movement, absolute amplitude, the morphology of the torso movement over the sampling period, and so on.

At 610, the process 600 includes performing additional respiratory measurements at one or more additional sets of conditions. For example, the system performs a second measurement session at a second requested breathing rate over a second sampling period. The second breathing rate may be higher or lower than the first breathing rate. The system may determine respiratory parameters to correspond to the respiratory parameters sensed during the first sampling period. For example, if a breathing power was determined over the first sampling period, the system may determine a breathing power over the second sampling period. Accordingly, the breathing powers from the sampling periods may be compared.

At 612, the process 600 includes determining a respiratory metric for a user based on the respiration parameters generated during the first sampling period and the second sampling period. A respiration metric may include an indication of whether a user is experiencing a respiratory condition such as a respiratory obstruction.

In some cases, the system may use the first respiratory parameters to determine a respiratory metric for a user. For example, one or more of the respiration parameters may be compared to one or more baseline metrics for the user, and the system can determine a metric, such as respiratory obstruction, based on comparing the first respiratory metric to baseline data obtained during an enrollment period. Additionally or alternatively, the system may evaluate a morphology of the respiratory parameter over the sampling period and determine a respiration metric based on the morphology of the signal. For example, the system can analyze how a user's chest movement changes over a sampling period to identify data that may be indicative of certain respiratory phenomenon such as dynamic hyperinflation where a user is inhaling more air than they are expelling when trying to breathe at a requested rate. These respiratory phenomena may be used to determine whether a user is experiencing an obstruction. In other cases, the phase lag of the user's chest movement compared to a requested breathing rate, and/or baseline data, may be used to determine a respiration metric.

In other embodiments, the first respiration parameter generated from the first sampling period may be compared to the second respiration parameter generated during the second respiration period to determine a respiration metric. For example, a drop in breathing power may be used to determine whether a user is experiencing a respiratory obstruction.

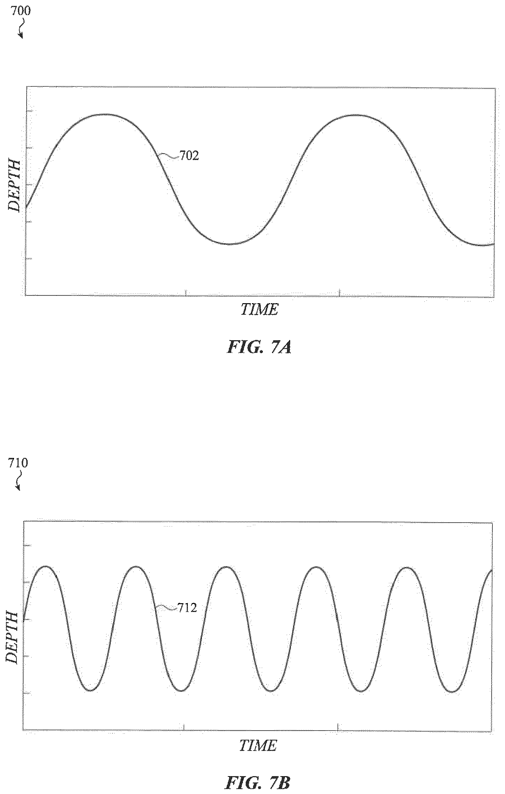

FIGs. 7A and 7B show examples of chest movement data measured at two different breathing rates. The chest movement data can include chest depth measurements taken over the respective sampling period, which include depth changes over time. The chest movement data is shown as a graph that includes depth measurements over time, for the sake of illustration. Although as implemented, this data may be processed by a computer system, as described herein, and be stored and processed in computer readable formats such as binary data.

FIG. 7A shows a first set of chest depth measurements 702 taken at a first respiration rate during a first sampling period 700 and FIG. 7B shows a second set of chest depth measurements 712 taken at a second respiration rate during a second sampling period 710. The second respiration rate is faster than the first respiration rate. In some cases, a first respiration parameter can include determining a first breathing power over the first sampling period 700. The first breathing power can indicate the amount of chest movement for the first sampling period 700 and be determined from the area of the chest depth measurement curve. The second respiration parameter can include determining a breathing power over the second sampling period 710. The second breathing power can be indicated as the amount of chest movement for the second sampling period 710 and be determined from the area of the chest depth measurement curve. A comparison of the first breathing power to the second breathing power can be used to determine a level of respiratory function of a user, as described herein.

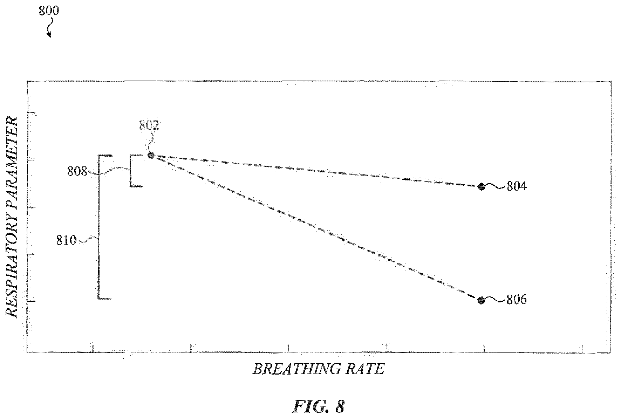

FIG. 8. shows an example power graph 800 for determining a breathing metric for a user based on first and second respiratory parameters determined during different sampling periods. The example power graph 800 includes breathing power parameters generated for different breathing rates. For example, a first breathing power 802 was determined for a first breathing rate during a first sampling period. A second breathing power 804 may correspond to a second breathing rate and be indicative of an individual that is not experiencing a respiratory obstruction. A third breathing power 806 may correspond to the second breathing rate and be indicative of an individual that is experiencing a respiratory obstruction.

In some cases, the respiration metric may be determined by comparing the breathing power at the first breathing rate to the second breathing power at the second breathing rate. There may be a change in breathing power between the first breathing rate and the second breathing rate that is associated with unobstructed breathing. This change can include a decrease in breathing power, an increase in breathing power, or no change in breathing power between the different rates. Accordingly, an unobstructed user may have a first change 808 in breathing power that is below a defined threshold. An obstructed user may have a larger change in breathing power, such as a large decrease in breathing power between the first breathing rate and the second breathing rate. Accordingly, an obstructed user may have a second change 810 that is greater than a defined threshold. As described herein, the obstruction threshold may be based on a variety of factors including demographic data for a user such as age, gender, height, weight, and so on. The system may determine the respiration metric, such as whether a user is experiencing a respiratory obstruction, based on determining a change in breathing power between breathing rates.

Additionally or alternatively, the system may determine a severity of an obstruction based on the decrease in the respiration parameter between different breathing rates. For example, when breathing power is used as a respiration metric, the system may define multiple threshold ranges each associated with a different obstruction severity. The threshold range that the change in breathing power falls within can be used to assign a severity of obstruction to a user.

The use of breathing power is provided as an example parameter that can be used to determine a respiratory metric for a user. However, other parameters can be used to determine one or more respiratory metrics. For example, other respiratory parameters can be based on changes in amplitude of a user's chest depth, breathing depth morphology including chest movement that indicates events such as dynamic hyperinflation, respiration lag behind the requested breathing rate, decreases in breathing power over a sampling period, and so on.

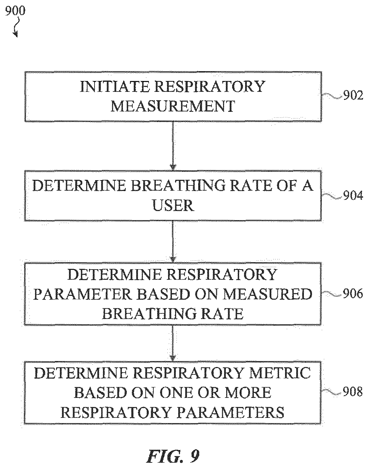

FIG. 9 shows an example process 900 for determining a respiratory parameter based on differences between a user's actual breathing rate and a requested breathing rate. The process 900 can be performed by the respiratory sensing systems described herein such as a system 100. In some cases, a user's actual breathing rate may deviate from the requested breathing rate. For this reason, in some embodiments, the system may measure a user's actual breathing rate and determine one or more respiration parameters for the user based on the measured breathing rate. In some cases, the respiration parameter determined based on the user's actual breathing rate may be different than a respiration parameter determined based on the requested rate.

At 902, the process 900 includes initiating a respiratory measurement for a first breathing rate. An electronic device instructs a user to breathe at a first breathing rate during a first sampling period, as described herein.

At 904, the process 900 can include determining an actual breathing rate of the user at the requested breathing rate. In some cases, the user's breathing rate can be determined from the chest depth measurements. Additionally or alternatively, other sensors can be used to measure a user's breathing rate such a wearable motion sensor, a microphone that records breathing sounds, and/or the like. In some cases, the user's measured breathing rate may deviate from the requested breathing rate. In some cases, the system (e.g., electronic device) may output one or more instructions for the user to adjust their breathing rate to match the requested rate. In other cases, the system may record the actual breathing rate and save this data along with the chest depth measurements and/or other respiration data obtained during the sampling period.

At 906, the process 900 can include determining a respiratory parameter based on the user's measured breathing rate. For example, if the user's breathing rate is slower than the requested breathing rate, the system may determine the user's breathing power may be adjusted to take into account the user's slower breathing rate. In some cases, the difference between the requested breathing rate and the user's measured breathing rate may used to generate a respiration parameter such as a breathing lag, which may indicate whether a user is struggling to breathe at the requested rate.

At 908, the process 900 can include determining a respiratory metric for the user based on the respiratory parameters that are generated from a user's measured breathing rate. For example, a breathing lag and/or change in breathing lag over a sampling period may be indicative of a breathing obstruction. Accordingly, the breathing lag may be measured and used to determine whether a user is experiencing a respiratory obstruction.

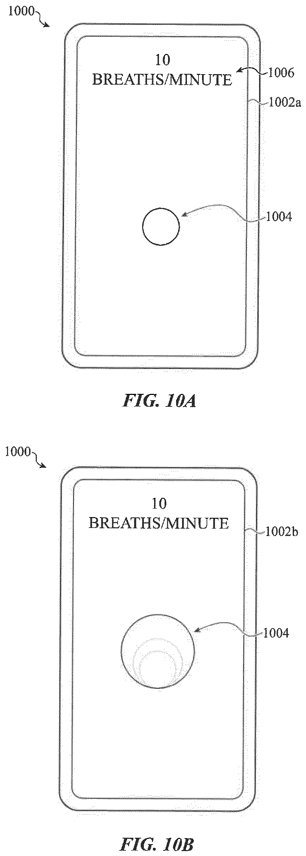

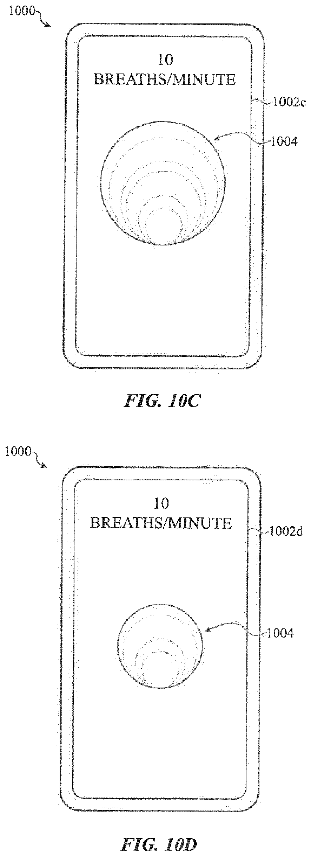

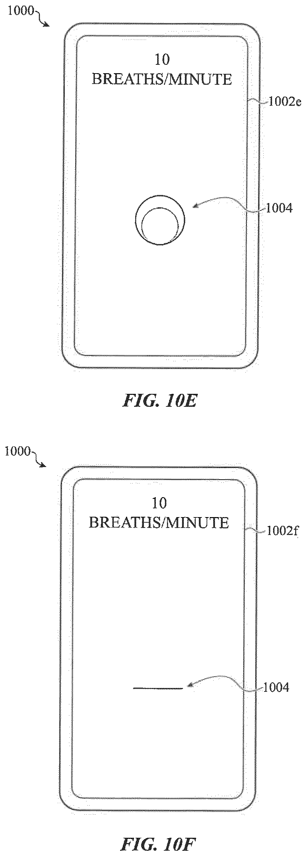

FIGs. 10A-10F show example outputs from an electronic device 1000 that can be used to guide a user through a requested breathing profile. The electronic device 1000 can include a display 1002 that can provide visual outputs 1004 to a user. In response to initiating a respiratory measurement session, the system may request a user to perform a guided breathing exercise. In some cases, the guided breathing exercise can include requesting the user to breathe at one or more constant breathing rates, which can each correspond to a different sampling period, as described herein. In other cases, the guided breathing exercise can include instructing the user to breathe at their natural breathing rate, performing dynamic breathing exercises, such as an increase and/or decrease in breathing rate over a sampling period, instructing a user to hold their breath for a period, and/or other breathing profiles.

In some cases, the electronic device 1000 can provide visual outputs 1004 that guide the user through a breathing exercise. For example, FIGs. 10A-10F illustrate a series of display instances 1002a-1002f that provide visual feedback of how the user should be breathing. For example a first visual output 1004 can dynamically adjust to show the instructed breathing rate to the user. In the illustrated example, the first visual output 1004 can be an expanding figure that expands and/or contracts at the desired breathing rate. For example, the first display instance 1002a (shown in FIG. 10A) may be displayed at the beginning of the breathing cycle, a subsequent display instance 1002c (shown in FIG. 10C) may be displayed at a maximum inhale time of the breathing cycle, and another display instance 1002f (shown in FIG. 10F) may be displayed at a maximum exhale time of the breathing cycle. Accordingly, a user may be able to breathe in unison with the visual output to achieve the requested breathing rate. Additionally or alternatively, the display instances 1002 may have additional visual outputs such as a second visual output 1006 (one of which is labeled for clarity) that indicate the desired breathing rate of the user.

The first visual output 1004 can change (e.g., expand and contract) at the desired breathing rate. For a first requested breathing rate, the first visual output 1004 can expand and contract based on the timing of a breathing cycle for that first rate. For a second requested breathing rate, the first visual output that can change to expand and contract based on the timing of a breathing cycle for the second rate. In cases of dynamically changing breathing rates, the first visual output 1004 and/or the second visual output 1006 can update in accordance with the change in the breathing rate.

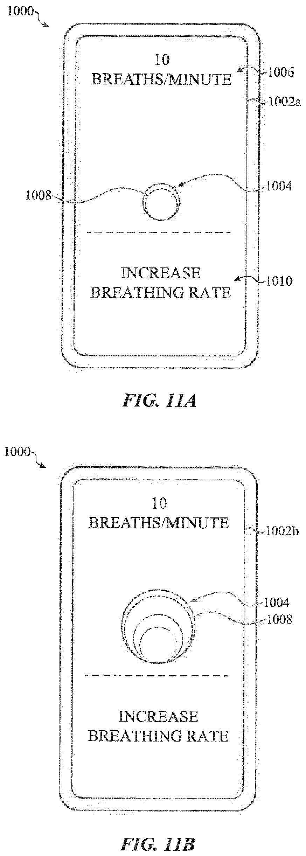

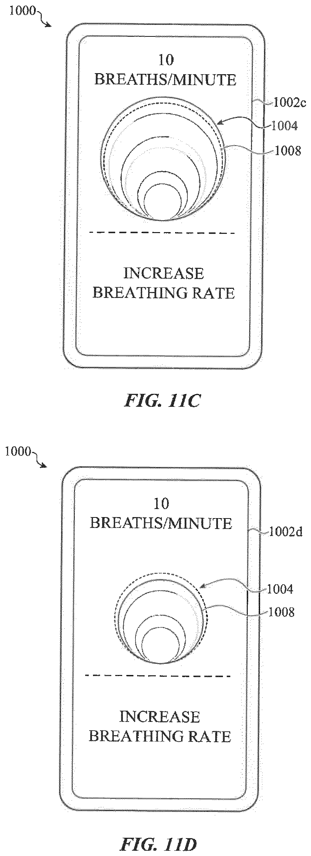

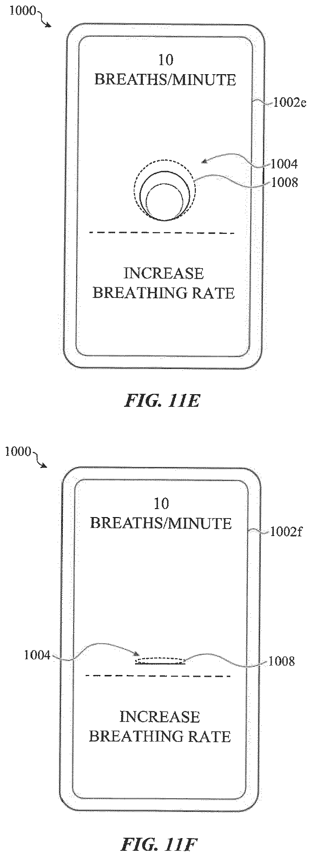

FIGs. 11A-11F show examples of a user output from an electronic device 1000 that can be used to guide a user through a requested breathing profile and provide user feedback. For example, the display instances 1002 can include a third visual output 1008 that indicates the user's actual breathing rate. In this example, the user is breathing slower than the requested rate, and the third visual output 1008 can indicate this by lagging behind the first visual output 1004, which expands and contracts at the requested rate. Additionally or alternatively, the system may provide a fourth visual output 1010 (one of which is labeled for clarity) that provides instructions to the user on how to change their breathing rate (e.g., increase their breathing rate). Accordingly, the system may provide real-time feedback to the user to help the user achieve/match the requested breathing rate.

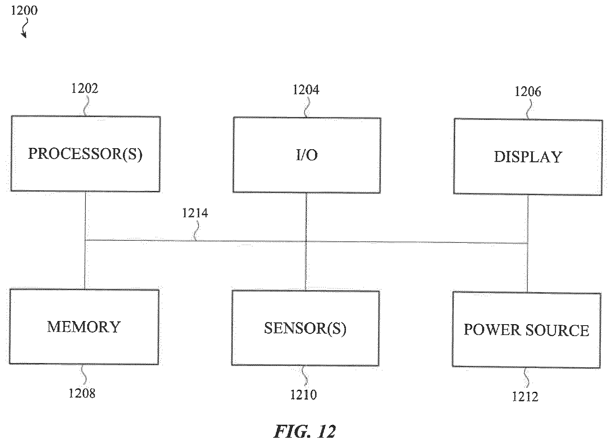

FIG. 12 is an example block diagram of a respiratory monitoring system 1200, which can take the form of any of the devices as described with references to FIGs. 1-11. The respiratory monitoring system 1200 can include a processor 1202, an input/output (I/O) mechanism 1204 (e.g., wired or wireless communications interfaces), a display 1206, memory 1208, sensors 1210 (e.g., physiological sensors such as those described herein), and a power source 1212 (e.g., a rechargeable battery). The processor 1202 can control some or all of the operations of the respiratory monitoring system 1200. The processor 1202 can communicate, either directly or indirectly, with some or all of the components of the respiratory monitoring system 1200. For example, a system bus or other communication mechanism 1214 can provide communication between the processor 1202, the I/O mechanism 1204, the memory 1208, the sensors 1210, and the power source 1212.

The processor 1202 can be implemented as any electronic device capable of processing, receiving, or transmitting data or instructions. For example, the processor 1202 can be a microprocessor, a central processing unit (CPU), an application-specific integrated circuit (ASIC), a digital signal processor (DSP), or combinations of such devices. As described herein, the term "processor" is meant to encompass a single processor or processing unit, multiple processors, multiple processing units, or other suitable computing element or elements. The processing unit can be programmed to perform the various aspects of the systems described herein.

It should be noted that the components of the respiratory monitoring system 1200 can be controlled by multiple processors. For example, select components of the respiratory monitoring system 1200 (e.g., a sensor 1210) may be controlled by a first processor and other components of the respiratory monitoring system 1200 (e.g., the I/O 1204) may be controlled by a second processor, where the first and second processors may or may not be in communication with each other.

The I/O device 1204 can transmit and/or receive data from a user or another electronic device. An I/O device can transmit electronic signals via a communications network, such as a wireless and/or wired network connection. Examples of wireless and wired network connections include, but are not limited to, cellular, Wi-Fi, Bluetooth, IR, and Ethernet connections. In some cases, the I/O device 1204 can communicate with an external electronic device, such as a smartphone, electronic device, or other portable electronic device, as described here.

The respiratory monitoring system may optionally include a display 1206 such as a liquid-crystal display (LCD), an organic light emitting diode (OLED) display, a light emitting diode (LED) display, or the like. If the display 1206 is an LCD, the display 1206 may also include a backlight component that can be controlled to provide variable levels of display brightness. If the display 1206 is an OLED or LED type display, the brightness of the display 1206 may be controlled by modifying the electrical signals that are provided to display elements. The display 1206 may correspond to any of the displays shown or described herein.

The memory 1208 can store electronic data that can be used by the respiratory monitoring system 1200. For example, the memory 1208 can store electrical data or content such as, for example, audio and video files, documents and applications, device settings and user preferences, timing signals, control signals, and data structures or databases. The memory 1208 can be configured as any type of memory. By way of example only, the memory 1208 can be implemented as random access memory, read-only memory, Flash memory, removable memory, other types of storage elements, or combinations of such devices.

The respiratory monitoring system 1200 may also include one or more sensors 1210 positioned almost anywhere on the respiratory monitoring system 1200. The sensor(s) 1210 can be configured to sense one or more types of parameters, such as but not limited to, pressure, light, touch, heat, movement, relative motion, biometric data (e.g., biological parameters), and so on. For example, the sensor(s) 1210 may include a heat sensor, a position sensor, a light or optical sensor, an accelerometer, a pressure transducer, a gyroscope, a magnetometer, a health monitoring sensor, and so on. Additionally, the one or more sensors 1210 can utilize any suitable sensing technology, including, but not limited to, capacitive, ultrasonic, resistive, optical, ultrasound, piezoelectric, and thermal sensing technology.

The power source 1212 can be implemented with any device capable of providing energy to the respiratory monitoring system 1200. For example, the power source 1212 may be one or more batteries or rechargeable batteries. Additionally or alternatively, the power source 1212 can be a power connector or power cord that connects the respiratory monitoring system 1200 to another power source, such as a wall outlet.

As described above, one aspect of the present technology is monitoring and managing physiological conditions of a user such as asthmatic events and the like. The present disclosure contemplates that in some instances this gathered data may include personal information data that uniquely identifies or can be used to contact or locate a specific person. Such personal information data can include demographic data, location-based data, telephone numbers, email addresses, Twitter IDs (or other social media aliases or handles), home addresses, data or records relating to a user's health or level of fitness (e.g., vital signs measurements, medication information, exercise information), date of birth, or any other identifying or personal information.

The present disclosure recognizes that the use of such personal information data, in the present technology, can be used to the benefit of users. For example, the personal information data can be used to provide haptic or audiovisual outputs that are tailored to the user. Further, other uses for personal information data that benefit the user are also contemplated by the present disclosure. For instance, health and fitness data may be used to provide insights into a user's general wellness or may be used as positive feedback to individuals using technology to pursue wellness goals.

The present disclosure contemplates that the entities responsible for the collection, analysis, disclosure, transfer, storage, or other use of such personal information data will comply with well-established privacy policies and/or privacy practices. In particular, such entities should implement and consistently use privacy policies and practices that are generally recognized as meeting or exceeding industry or governmental requirements for maintaining the privacy and security of personal information data. Such policies should be easily accessible by users and should be updated as the collection and/or use of data changes. Personal information from users should be collected for legitimate and reasonable uses of the entity and not shared or sold outside of those legitimate uses. Further, such collection/sharing should occur after receiving the informed consent of the users. Additionally, such entities should consider taking any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices. In addition, policies and practices should be adapted for the particular types of personal information data being collected and/or accessed and revised to adhere to applicable laws and standards, including jurisdiction-specific considerations. For instance, in the US, collection of or access to certain health data may be governed by federal and/or state laws, such as the Health Insurance Portability and Accountability Act ("HIPAA"); whereas health data in other countries may be subject to other regulations and policies and should be handled accordingly. Hence different privacy practices should be maintained for different personal data types in each country.

Despite the foregoing, the present disclosure also contemplates embodiments in which users selectively block the use of, or access to, personal information data. That is, the present disclosure contemplates that hardware and/or software elements can be provided to prevent or block access to such personal information data. For example, in the case of determining spatial parameters, the present technology can be configured to allow users to select to "opt in" or "opt out" of participation in the collection of personal information data during registration for services or anytime thereafter. In addition to providing "opt in" and "opt out" options, the present disclosure contemplates providing notifications relating to the access or use of personal information. For instance, a user may be notified upon downloading an app that their personal information data will be accessed and then reminded again just before personal information data is accessed by the app.

Moreover, it is the intent of the present disclosure that personal information data should be managed and handled in a way to minimize risks of unintentional or unauthorized access or use. Risk can be minimized by limiting the collection of data and deleting data once it is no longer needed. In addition, and when applicable, including in certain health related applications, data de-identification can be used to protect a user's privacy. De-identification may be facilitated, when appropriate, by removing specific identifiers (e.g., date of birth, etc.), controlling the amount or specificity of data stored (e.g., collecting location data at a city level rather than at an address level), controlling how data is stored (e.g., aggregating data across users), and/or other methods.

Therefore, although the present disclosure broadly covers use of personal information data to implement one or more various disclosed embodiments, the present disclosure also contemplates that the various embodiments can also be implemented without the need for accessing such personal information data. That is, the various embodiments of the present technology are not rendered inoperable due to the lack of all or a portion of such personal information data. For example, haptic outputs may be provided based on non-personal information data or a bare minimum amount of personal information, such as events or states at the device associated with a user, other non-personal information, or publicly available information.

The foregoing description, for purposes of explanation, used specific nomenclature to provide a thorough understanding of the described embodiments. The foregoing descriptions of the specific embodiments described herein are presented for purposes of illustration and description. They are not targeted to be exhaustive or to limit the embodiments to the precise forms disclosed. The invention is defined by the appended independent claims.

## Claims

1. A system for measuring respiratory function of a user, the system comprising:
an optical sensing unit configured to:
identify a torso of the user;
detect movement of the torso with respect to the optical sensing unit; and
output one or more signals indicative of the movement of the torso;
an electronic device configured to provide a first request for the user to breathe at a first rate during a first time period and a second request for the user to breathe at a second rate during a second time period; and
a processing unit programmed to:
receive first signals generated by the optical sensing unit during the first time period based on the movement of the torso and determine a first respiration parameter using the first signals;
receive second signals generated by the optical sensing unit during the second time period based on the movement of the torso and determine a second respiration parameter using the second signals; and
determine a level of respiratory function based on the first respiration parameter and the second respiration parameter.

2. The system of claim 1, wherein the optical sensing unit comprises:
a camera that is configured to identify the torso of the user;
a depth sensor that is configured to detect changes in depth of the torso; and
detecting the movement of the torso comprises detecting the changes in depth of the torso with respect to the optical sensing unit.

3. The system of claim 1, wherein the optical sensing unit is further configured to:
identify one or more sampling regions along the torso of the user; and
detect movement at each of the one or more sampling regions.

4. The system of claim 3, wherein the processing unit is configured to select at least one sampling region and determine the first respiration parameter and the second respiration parameter using signals of the one or more signals corresponding to the selected at least one sampling region.

5. The system of claim 1, wherein:
the first rate is slower than the second rate;
the first and second respiration parameters each comprise a corresponding signal power; and
the level of respiratory function is based on an amount of decrease in the corresponding signal power between the first rate and the second rate.

6. The system of claim 1, wherein the processing unit is further configured to:
determine a first breathing rate of the user in response to the first request, the first breathing rate based on the first signals generated during the first time period; and
determine a second breathing rate of the user in response to the second request, the second breathing rate based on second signals generated during the second time period;
wherein determining the level of respiratory function comprising using the first and second breathing rates.

7. The system of claim 1, wherein:
the electronic device comprises at least one of a display and a speaker; and
the first and second requests each comprise at least one of visual output that is displayed on the display and an audio output from the speaker.

8. The system of claim 1, wherein the processing unit is further programmed to:
generate a body model of the user; and
use the body model to determine the first and second respiration parameters.

9. The system of claim 1, wherein:
the optical sensing unit comprises a motion sensor;
the optical sensing unit detects the movement of the torso by measuring distances traveled by the torso of the user while the motion sensor is coupled to the torso; and
the processing unit is programmed to determine the first respiration parameter based on the distances traveled by the torso of the user measured while the motion sensor is coupled to the torso.

10. The system of claim 1, wherein:
the optical sensing unit comprises:
a first device; and
a second device configured to track movement of the first device and measure a first distance between the first device and the second device; and
the optical sensing unit detects the movement of the torso by measuring the first distance with the second device while the first device is coupled to the torso of the user.

11. The system of claim 10, wherein:
the first and second devices each comprise one or more antennas that are operable to determine the first distance.

12. The system of claim 10, wherein:
the second device comprises an optical sensor that is configured to measure the first distance.

13. A method for measuring respiratory function of a user, the method comprising:
outputting, from an electronic device, a first request for the user to breathe at a first rate for a first time period;
obtaining, by an optical sensing unit, a first set of respiratory measurements during the first time period, the first set of respiratory measurements comprising measurements corresponding to movement of a torso of the user;
outputting, from the electronic device, a second request for the user to breathe at a second rate for a second time period;
obtaining, by the optical sensing unit, a second set of respiratory measurements during the second time period, the second set of respiratory measurements comprising measurements corresponding to the movement of the torso of the user; and
determining, by a processing unit, a respiratory metric for the user based on the first and second sets of respiratory measurements.

14. The method of claim 13, further comprising:
in response to outputting the first request, detecting by the optical sensing unit a first breathing rate of the user;
in response to the first breathing rate satisfying a first criteria, obtaining the first set of respiratory measurements;
in response to outputting the second request, detecting by the optical sensing unit a second breathing rate of the user; and
in response to the second breathing rate satisfying a second criteria, obtaining the second set of respiratory measurements.

15. The method of claim 13, further comprising:
determining, by the processing unit, a first power metric based on the first set of respiratory measurements; and
determining, by the processing unit, a second power metric based on the second set of respiratory measurements, wherein determining the respiratory metric comprises determining an obstruction metric for the user based on the first and second power metrics.

## Patentansprüche

1. System zum Messen der Atmungsfunktion eines Benutzers, wobei das System Folgendes aufweist:
eine optische Erfassungseinheit, die konfiguriert ist zum:
Identifizieren eines Torsos des Benutzers;
Detektieren einer Bewegung des Torsos in Bezug auf die optische Erfassungseinheit; und
Ausgeben eines oder mehrerer Signale, die die Bewegung des Torsos anzeigen;
eine elektronische Vorrichtung, die konfiguriert ist zum Vorsehen einer ersten Anforderung für den Benutzer, mit einer ersten Rate während einer ersten Zeitperiode zu atmen, und einer zweiten Anforderung für den Benutzer, mit einer zweiten Rate während einer zweiten Zeitperiode zu atmen; und
eine Verarbeitungseinheit, die programmiert ist zum:
Empfangen erster Signale, die durch die optische Erfassungseinheit während der ersten Zeitperiode basierend auf der Bewegung des Torsos erzeugt werden, und Bestimmen eines ersten Atmungsparameters unter Verwendung der ersten Signale;
Empfangen zweiter Signale, die durch die optische Erfassungseinheit während der zweiten Zeitperiode basierend auf der Bewegung des Torsos erzeugt werden, und Bestimmen eines zweiten Atmungsparameters unter Verwendung der zweiten Signale; und
Bestimmen eines Niveaus der Atmungsfunktion basierend auf dem ersten Atmungsparameter und dem zweiten Atmungsparameter.

2. System nach Anspruch 1, wobei die optische Erfassungseinheit Folgendes aufweist:
eine Kamera, die konfiguriert ist zum Identifizieren des Torsos des Benutzers;
einen Tiefensensor, der konfiguriert ist zum Detektieren von Änderungen der Tiefe des Torsos; und
wobei das Detektieren der Bewegung des Torsos das Detektieren der Änderungen der Tiefe des Torsos in Bezug auf die optische Erfassungseinheit aufweist.

3. System nach Anspruch 1, wobei die optische Erfassungseinheit ferner konfiguriert ist zum: Identifizieren eines oder mehrerer Abtastbereiche entlang des Torsos des Benutzers; und
Detektieren einer Bewegung an jedem des einen oder der mehreren Abtastbereiche.

4. System nach Anspruch 3, wobei die Verarbeitungseinheit konfiguriert ist zum Auswählen mindestens eines Abtastbereichs und Bestimmen des ersten Atmungsparameters und des zweiten Atmungsparameters unter Verwendung von Signalen des einen oder der mehreren Signale, die dem ausgewählten mindestens einen Abtastbereich entsprechen.

5. System nach Anspruch 1, wobei:
die erste Rate langsamer als die zweite Rate ist;
der erste und der zweite Atmungsparameter jeweils eine entsprechende Signalleistung aufweisen; und
das Niveau der Atmungsfunktion auf einem Betrag der Abnahme der entsprechenden Signalleistung zwischen der ersten Rate und der zweiten Rate basiert.

6. System nach Anspruch 1, wobei die Verarbeitungseinheit ferner konfiguriert ist zum:
Bestimmen einer ersten Atmungsrate des Benutzers als Reaktion auf die erste Anforderung, wobei die erste Atmungsrate auf den ersten Signalen basiert, die während der ersten Zeitperiode erzeugt werden; und
Bestimmen einer zweiten Atmungsrate des Benutzers als Reaktion auf die zweite Anforderung, wobei die zweite Atmungsrate auf zweiten Signalen basiert, die während der zweiten Zeitperiode erzeugt werden;
wobei das Bestimmen des Niveaus der Atmungsfunktion das Verwenden der ersten und der zweiten Atmungsrate aufweist.

7. System nach Anspruch 1, wobei:
die elektronische Vorrichtung eine Anzeige und/oder einen Lautsprecher aufweist; und
die erste und die zweite Anforderung jeweils eine visuelle Ausgabe, die auf der Anzeige angezeigt wird, und/oder eine Audioausgabe von dem Lautsprecher aufweisen.

8. System nach Anspruch 1, wobei die Verarbeitungseinheit ferner programmiert ist zum:
Erzeugen eines Körpermodells des Benutzers; und
Verwenden des Körpermodells, um den ersten und den zweiten Atmungsparameter zu bestimmen.

9. System nach Anspruch 1, wobei:
die optische Erfassungseinheit einen Bewegungssensor aufweist;
die optische Erfassungseinheit die Bewegung des Torsos durch Messen von Entfernungen detektiert, die von dem Torso des Benutzers zurückgelegt werden, während der Bewegungssensor mit dem Torso gekoppelt ist; und
die Verarbeitungseinheit programmiert ist zum Bestimmen des ersten Atmungsparameters basierend auf den Entfernungen, die von dem Torso des Benutzers zurückgelegt werden, die gemessen werden, während der Bewegungssensor mit dem Torso gekoppelt ist.

10. System nach Anspruch 1, wobei:
die optische Erfassungseinheit Folgendes aufweist:
eine erste Vorrichtung; und
eine zweite Vorrichtung, die konfiguriert ist zum Verfolgen der Bewegung der ersten Vorrichtung und zum Messen einer ersten Entfernung zwischen der ersten Vorrichtung und der zweiten Vorrichtung; und
die optische Erfassungseinheit die Bewegung des Torsos durch Messen der ersten Entfernung mit der zweiten Vorrichtung detektiert, während die erste Vorrichtung mit dem Torso des Benutzers gekoppelt ist.

11. System nach Anspruch 10, wobei:
die erste und die zweite Vorrichtung jeweils eine oder mehrere Antennen aufweisen, die betreibbar sind zum Bestimmen der ersten Entfernung.

12. System nach Anspruch 10, wobei:
die zweite Vorrichtung einen optischen Sensor aufweist, der konfiguriert ist zum Messen der ersten Entfernung.

13. Verfahren zum Messen der Atmungsfunktion eines Benutzers, wobei das Verfahren Folgendes aufweist:
Ausgeben, von einer elektronischen Vorrichtung, einer ersten Anforderung für den Benutzer, mit einer ersten Rate für eine erste Zeitperiode zu atmen;
Erhalten, durch eine optische Erfassungseinheit, eines ersten Satzes von Atmungsmessungen während der ersten Zeitperiode, wobei der erste Satz von Atmungsmessungen Messungen entsprechend der Bewegung eines Torsos des Benutzers aufweist;
Ausgeben, von der elektronischen Vorrichtung, einer zweiten Anforderung für den Benutzer, mit einer zweiten Rate für eine zweite Zeitperiode zu atmen;
Erhalten, durch die optische Erfassungseinheit, eines zweiten Satzes von Atmungsmessungen während der zweiten Zeitperiode, wobei der zweite Satz von Atmungsmessungen Messungen entsprechend der Bewegung des Torsos des Benutzers aufweist; und
Bestimmen, durch eine Verarbeitungseinheit, einer Atmungsmetrik für den Benutzer basierend auf dem ersten und dem zweiten Satz von Atmungsmessungen.

14. Verfahren nach Anspruch 13, das ferner Folgendes aufweist:
als Reaktion auf das Ausgeben der ersten Anforderung, Detektieren, durch die optische Erfassungseinheit, einer ersten Atmungsrate des Benutzers;
als Reaktion darauf, dass die erste Atmungsrate ein erstes Kriterium erfüllt, Erhalten des ersten Satzes von Atmungsmessungen;
als Reaktion auf das Ausgeben der zweiten Anforderung, Detektieren, durch die optische Erfassungseinheit, einer zweiten Atmungsrate des Benutzers; und
als Reaktion darauf, dass die zweite Atmungsrate ein zweites Kriterium erfüllt, Erhalten des zweiten Satzes von Atmungsmessungen.

15. Verfahren nach Anspruch 13, das ferner Folgendes aufweist:
Bestimmen, durch die Verarbeitungseinheit, einer ersten Leistungsmetrik basierend auf dem ersten Satz von Atmungsmessungen; und
Bestimmen, durch die Verarbeitungseinheit, einer zweiten Leistungsmetrik basierend auf dem zweiten Satz von Atmungsmessungen, wobei das Bestimmen der Atmungsmetrik das Bestimmen einer Obstruktionsmetrik für den Benutzer basierend auf der ersten und der zweiten Leistungsmetrik aufweist.

## Revendications

1. Un système de mesure d'une fonction respiratoire d'un utilisateur, le système comprenant :
une unité de détection optique configurée pour :
identifier le torse de l'utilisateur ;
détecter un mouvement du torse par rapport à l'unité de détection optique ; et
délivrer un ou plusieurs signaux représentatifs du mouvement du torse ;
un dispositif électronique configuré pour produire une première requête pour que l'utilisateur respire à un premier rythme pendant un premier laps de temps, et une seconde requête pour que l'utilisateur respire à un second rythme pendant un second laps de temps ; et
une unité de traitement programmée pour :
recevoir des premiers signaux générés pendant le premier laps de temps par l'unité de détection optique sur la base du mouvement du torse, et déterminer un premier paramètre de respiration en utilisant les premiers signaux ;
recevoir des seconds signaux générés pendant le second laps de temps par l'unité de détection optique sur la base du mouvement du torse, et déterminer un second paramètre de respiration en utilisant les seconds signaux ; et
déterminer un niveau de fonction respiratoire sur la base du premier paramètre de respiration et le second paramètre de respiration.

2. Le système de la revendication 1, dans lequel l'unité de détection optique comprend :
une caméra qui est configurée pour identifier le torse de l'utilisateur ;
un capteur de profondeur qui est configuré pour détecter les changements de profondeur du torse ; et
la détection du mouvement du torse comprend la détection des changements de profondeur du torse par rapport à l'unité de détection optique.

3. Le système de la revendication 1, dans lequel l'unité de détection optique est en outre configurée pour :
identifier une ou plusieurs régions d'échantillonnage le long du torse de l'utilisateur ; et
détecter le mouvement au niveau de chacune des une ou plusieurs régions d'échantillonnage.

4. Le système de la revendication 3, dans lequel l'unité de traitement est configurée pour sélectionner au moins une région d'échantillonnage et déterminer le premier paramètre de respiration et le second paramètre de respiration en utilisant des signaux, d'entre les un ou plusieurs signaux, qui correspondent à l'au moins une région d'échantillonnage sélectionnée.

5. Le système de la revendication 1, dans lequel :
le premier rythme est plus lent que le second rythme ;
le premier et le second paramètre de respiration comprennent chacun une puissance correspondante de signal ; et
le niveau de la fonction respiratoire est basé sur une quantité de diminution de la puissance de signal correspondante, entre le premier rythme et le second rythme.

6. Le système de la revendication 1, dans lequel l'unité de détection optique est en outre configurée pour :
déterminer un premier rythme de respiration de l'utilisateur en réponse à la première requête, le premier rythme de respiration étant basé sur les premiers signaux générés pendant le premier laps de temps ; et
déterminer un second rythme de respiration de l'utilisateur en réponse à la seconde requête, le second rythme de respiration étant basé sur les seconds signaux générés pendant le second laps de temps ;
dans lequel la détermination du niveau de la fonction respiratoire comprend l'utilisation du premier et du second rythme de respiration.

7. Le système de la revendication 1, dans lequel :
le dispositif électronique comprend au moins l'un d'entre un afficheur et un haut-parleur ; et
la première et la seconde requête comprennent chacune au moins l'une d'entre une sortie visuelle qui est affichée sur l'afficheur, et une sortie audio depuis le haut-parleur.

8. Le système de la revendication 1, dans lequel l'unité de traitement est en outre programmée pour :
générer un modèle corporel de l'utilisateur ; et
utiliser le modèle corporel pour déterminer le premier et le second paramètre de respiration.

9. Le système de la revendication 1, dans lequel :
l'unité de détection optique comprend un capteur de déplacement ;
l'unité de détection optique détecte le mouvement du torse par mesure des distances parcourues par le torse de l'utilisateur lorsque le capteur de déplacement est couplé au torse ; et
l'unité de traitement est programmée pour déterminer le premier paramètre de respiration sur la base des distances parcourues par le torse de l'utilisateur, mesurées pendant que le capteur de déplacement est couplé au torse.

10. Le système de la revendication 1, dans lequel :
l'unité de détection optique comprend :
un premier dispositif ; et
un second dispositif configuré pour suivre le mouvement du premier dispositif et mesurer une première distance entre le premier dispositif et le second dispositif ; et
l'unité de détection optique détecte le mouvement du torse par mesure de la première distance avec le second dispositif lorsque le premier dispositif est couplé au torse de l'utilisateur.

11. Le système de la revendication 10, dans lequel :
le premier et le second dispositif comprennent chacun une ou plusieurs antennes qui peuvent être mises en œuvre pour déterminer la première distance.

12. Le système de la revendication 10, dans lequel :
le second dispositif comprend un capteur optique qui est configuré pour mesurer la première distance.

13. Un procédé de mesure d'une fonction respiratoire d'un utilisateur, le procédé comprenant :
la délivrance, depuis un dispositif électronique, d'une première requête pour que l'utilisateur respire à un premier rythme pendant un premier laps de temps ;
l'obtention, par une unité de détection optique, d'un premier ensemble de mesures respiratoires pendant le premier laps de temps, le premier ensemble de mesures respiratoires comprenant des mesures correspondant aux mouvements du torse de l'utilisateur ;
la délivrance, depuis le dispositif électronique, d'une seconde requête pour que l'utilisateur respire à un second rythme pendant un second laps de temps ;
l'obtention, par l'unité de détection optique, d'un second ensemble de mesures respiratoires pendant le second laps de temps, le second ensemble de mesures respiratoires comprenant des mesures correspondant aux mouvements du torse de l'utilisateur ; et
la détermination, par une unité de traitement, d'une métrique respiratoire pour l'utilisateur, sur la base du premier et du second ensemble de mesures respiratoires.

14. Le procédé de la revendication 13, comprenant en outre :
en réponse à la délivrance de la première requête, la détection par l'unité de détection optique d'un premier rythme de respiration de l'utilisateur ;
en réponse au fait que le premier rythme de respiration vérifie un premier critère, l'obtention du premier ensemble de mesures respiratoires ;
en réponse à la délivrance de la seconde requête, la détection par l'unité de détection optique d'un second rythme de respiration de l'utilisateur ; et
en réponse au fait que le second rythme de respiration vérifie un second critère, l'obtention du second ensemble de mesures respiratoires.

15. Le procédé de la revendication 13, comprenant en outre :
la détermination, par l'unité de traitement, d'une première métrique de puissance basée sur le premier ensemble de mesures respiratoires, et
la détermination, par l'unité de traitement, d'une seconde métrique de puissance basée sur le second ensemble de mesures respiratoires, la détermination de la mesure respiratoire comprenant la détermination d'une métrique d'obstruction pour l'utilisateur, sur la base de la première et de la seconde métrique de puissance.
